# EUROPEAN PATENT APPLICATION

(11) **EP 2 210 885 A1**
(43) Date of publication of application: **28.07.2010**
(21) Application number: 09000437.5
(22) Date of filing: 14.01.2009
(51) Int. Cl.: C07D 401/04, A61K 31/4545, A61P 29/00

(54) **Substituted heteroarylpiperidine derivatives as melanocortin-4 receptor modulators**

(71) Applicant: Santhera Pharmaceuticals (Schweiz) AG, 4410 Liestal (CH)
(72) Inventor: Herzner, Holger, 79595 Rümmingen (DE); Henneböhle, Marco, 79618 Rheinfelden (DE); Feurer, Achim, 79424 Auggen (DE); Nordhoff, Sonja, 4144 Arlesheim (CH); Soeberdt, Michael, 79618 Rheinfelden (DE)
(74) Representative: Grünecker, Kinkeldey, Stockmair & Schwanhäusser Anwaltssozietät

(57) **Abstract**

The present invention relates to substituted heteroarylpiperidine derivatives as melanocortin-4 receptor modulators, in particular as melanocortin 4 receptor antagonists. The antagonists are useful for the treatment of disorders and diseases such as cachexia induced by e.g. cancer, chronic kidney disease (CKD) or chronic heart failure (CHF), muscle wasting, anorexia induced by e.g. chemotherapy or radiotherapy, anorexia nervosa, amyotrophic lateral sclerosis (ALS), pain, neuropathic pain, anxiety and depression, nausea and emesis.

## Description

### Field of the Invention

The present invention relates to substituted heteroarylpiperidine derivatives as melanocortin-4 receptor modulators. Depending on the structure and the stereochemistry the compounds of the invention are selective antagonists of the human melanocortin-4 receptor (MC-4R). The antagonists are useful for the treatment of disorders and diseases such as cachexia induced by e.g. cancer, chronic kidney disease (CKD) or chronic heart failure (CHF), muscle wasting, anorexia induced by e.g. chemotherapy or radiotherapy, anorexia nervosa, amyotrophic lateral sclerosis (ALS), pain, neuropathic pain, anxiety and depression, nausea and emesis.

### Background of the Invention

Melanocortins (MCs) stem from pro-opiomelanocortin (POMC) via proteolytic cleavage. These peptides, adrenocorticotropic hormone (ACTH), α-melanocyte-stimulating hormone (α-MSH), β-MSH and γ-MSH, range in size from 12 to 39 amino acids. The most important endogenous agonist for central MC-4R activation appears to be the tridecapeptide α-MSH. Among MCs, it was reported that α-MSH acts as a neurotransmitter or neuromodulator in the brain. MC peptides, particularly α-MSH, have a wide range of effects on biological functions including feeding behavior, pigmentation and exocrine function. The biological effects of α-MSH are mediated by a sub-family of 7-transmembrane G-protein-coupled receptors, termed melanocortin receptors (MC-Rs). Activation of any of these MC-Rs results in stimulation of cAMP formation.

To date, five distinct types of receptor subtype for MC (MC-1 R to MC-5R) have been identified and these are expressed in different tissues.

MC-1 R was first found in melanocytes. Naturally occurring inactive variants of MC-1 R in animals were shown to lead to alterations in pigmentation and a subsequent lighter coat color by controlling the conversion of phaeomelanin to eumelanin through the control of tyrosinase. From these and other studies, it is evident that MC-1 R is an important regulator of melanin production and coat color in animals and skin color in humans.

The MC-2R is expressed in the adrenal gland representing the ACTH receptor. The MC-2R is not a receptor for α-MSH but is the receptor for the adrenocorticotropic hormone I (ACTH I).

The MC-3R is expressed in the brain (predominately located in the hypothalamus) and peripheral tissues like gut and placenta, and knock-out studies have revealed that the MC-3R may be responsible for alterations in feeding behavior, body weight and thermogenesis.

The MC-4R is primarily expressed in the brain. Overwhelming data support the role of MC-4R in energy homeostasis. Genetic knock-outs and pharmacologic manipulation of MC-4R in animals have shown that agonizing the MC-4R causes weight loss and antagonizing the MC-4R produces weight gain (A. Kask et al., "Selective antagonist for the melanocortin-4 receptor (HS014) increases food intake in free-feeding rats," Biochem. Biophys. Res. Commun., 245: 90-93 (1998)).

MC-5R is ubiquitously expressed in many peripheral tissues including white fat, placenta and a low level of expression is also observed in the brain. However its expression is greatest in exocrine glands. Genetic knock-out of this receptor in mice results in altered regulation of exocrine gland function, leading to changes in water repulsion and thermoregulation. MC-5R knockout mice also reveal reduced sebaceous gland lipid production (Chen et al., Cell, 91: 789-798 (1997)).

Attention has been focused on the study of MC-3R and MC-4R modulators and their use in treating body weight disorders, such as obesity and anorexia. However, evidence has shown that the MC peptides have potent physiological effects besides their role in regulating pigmentation, feeding behavior and exocrine function. In particular, α-MSH recently has been shown to induce a potent anti-inflammatory effect in both acute and chronic models of inflammation including inflammatory bowel-disease, renal ischemia/reperfusion injury and endotoxin-induced hepatitis. Administration of α-MSH in these models results in substantial reduction of inflammation-mediated tissue damage, a significant decrease in leukocyte infiltration and a dramatic reduction in elevated levels of cytokines and other mediators to near baseline levels. Recent studies have demonstrated that the anti-inflammatory actions of α-MSH are mediated by MC-1R. The mechanism by which agonism of MC-1R results in an anti-inflammatory response is likely through inhibition of the pro-inflammatory transcription activator, NF-κB. NF-κB is a pivotal component of the pro-inflammatory cascade, and its activation is a central event in initiating many inflammatory diseases. Additionally, anti-inflammatory actions of α-MSH may be, in part, mediated by agonism of MC-3R and/or MC-5R.

A specific single MC-R that may be targeted for the control of obesity has not yet been identified, although evidence has been presented that MC-4R signaling is important in mediating feeding behavior (S.Q. Giraudo et al., "Feeding effects of hypothalamic injection of melanocortin-4 receptor ligands," Brain Research, 80: 302-306 (1998)). Further evidence for the involvement of MC-Rs in obesity includes: 1) the agouti (A^{vy}) mouse which ectopically expresses an antagonist of the MC-1R, MC-3R and MC-4R is obese, indicating that blocking the action of these three MC-R's can lead to hyperphagia and metabolic disorders; 2) MC-4R knockout mice (D. Huszar et al., Cell, 88: 131-141 (1997)) recapitulate the phenotype of the agouti mouse and these mice are obese; 3) the cyclic heptapeptide melanotanin II (MT-II) (a non-selective MC-1R, -3R, -4R, and -5R agonist) injected intracerebroventricularly (ICV) in rodents, reduces food intake in several animal feeding models (NPY, ob/ob, agouti, fasted) while ICV injected SHU-9119 (MC-3R and 4R antagonist; MC-1 R and -5R agonist) reverses this effect and can induce hyperphagia; 4) chronic intraperitoneal treatment of Zucker fatty rats with an α-NDP-MSH derivative (HP-228) has been reported to activate MC-1R, -3R, -4R, and -5R and to attenuate food intake and body weight gain over a 12 week period (I. Corcos et al., "HP-228 is a potent agonist of melanocortin receptor-4 and significantly attenuates obesity and diabetes in Zucker fatty rats", Society for Neuroscience Abstracts, 23: 673 (1997)).

MC-4R appears to play a role in other physiological functions as well, namely controlling grooming behavior, erection and blood pressure. Erectile dysfunction denotes the medical condition of inability to achieve penile erection sufficient for successful intercourse. The term "impotence" is often employed to describe this prevalent condition. Synthetic melanocortin receptor agonists have been found to initiate erections in men with psychogenic erectile dysfunction (H. Wessells et al., "Synthetic Melanotropic Peptide Initiates Erections in Men With Psychogenic Erectile Dysfunction: Double-Blind, Placebo Controlled Crossover Study", J. Urol., 160: 389-393, (1998)). Activation of melanocortin receptors of the brain appears to cause normal stimulation of sexual arousal. Evidence for the involvement of MC-R in male and/or female sexual dysfunction is detailed in WO 00/74679.

Diabetes is a disease in which a mammal's ability to regulate glucose levels in the blood is impaired because the mammal has a reduced ability to convert glucose to glycogen for storage in muscle and liver cells. In Type I diabetes, this reduced ability to store glucose is caused by reduced insulin production. "Type II diabetes" or "Non-Insulin Dependent Diabetes Mellitus" (NIDDM) is the form of diabetes which is due to a profound resistance to insulin stimulating or regulatory effect on glucose and lipid metabolism in the main insulin-sensitive tissues, muscle, liver and adipose tissue. This resistance to insulin responsiveness results in insufficient insulin activation of glucose uptake, oxidation and storage in muscle, and inadequate insulin repression of lipolysis in adipose tissue and of glucose production and secretion in liver. When these cells become desensitized to insulin, the body tries to compensate by producing abnormally high levels of insulin and hyperinsulemia results. Hyperinsulemia is associated with hypertension and elevated body weight. Since insulin is involved in promoting the cellular uptake of glucose, amino acids and triglycerides from the blood by insulin sensitive cells, insulin insensitivity can result in elevated levels of triglycerides and LDL which are risk factors in cardiovascular diseases. The constellation of symptoms which includes hyperinsulemia combined with hypertension, elevated body weight, elevated triglycerides and elevated LDL, is known as Syndrome X. MC-4R agonists might be useful in the treatment of NIDDM and Syndrome X.

Among MC receptor subtypes, the MC4 receptor is also of interest in terms of the relationship to stress and the regulation of emotional behavior, as based on the following findings. Stress initiates a complex cascade of responses that include endocrine, biochemical and behavioral events. Many of these responses are initiated by release of corticotropin-releasing factor (CRF) (M.J. Owen and C.B. Nemeroff, "Physiology and pharmacology of corticotrophin releasing factor." Pharmacol. Rev. 43: 425-473 (1991)). In addition to activation of the brain CRF system, there are several lines of evidence that melanocortins (MCs), which stem from proopiomelanocortin by enzymatic processing, mediate important behavioral and biochemical responses to stress and, consequently, stress-induced disorders like anxiety and depression (Shigeyuki Chaki et al, "Anxiolytic-Like and Antidepressant-Like Activities of MCL0129 (1-[(S)-2-(4-Fluorophenyl)-2-(4-isopropylpiperadin-1-yl)ethyl]-4- [4-(2-methoxynaphthalen-1-yl)butyl]piperazine), a Novel and Potent Nonpeptide Antagonist of the Melanocortin-4 Receptor", J. Pharm. Exp. Ther. 304(2), 818-826 (2003)).

Chronic diseases, such as malignant tumors or infections, are frequently associated with cachexia resulting from a combination of a decrease in appetite and a loss of lean body mass. Extensive loss of lean body mass is often triggered by an inflammatory process and is usually associated with increased plasma levels of cytokines (e.g. TNF-α), which increase the production of α-MSH in the brain. Activation of MC4 receptors in the hypothalamus by α-MSH reduces appetite and increases energy expenditure.

Experimental evidence in tumor bearing mice suggests that cachexia can be prevented or reversed by genetic MC4 receptor knockout or MC4 receptor blockade. The increased body weight in the treated mice is attributable to a larger amount of lean body mass, which mainly consists of skeletal muscle (D.L. Marks et al. "Role of the central melanocortin system in cachexia." Cancer Res. 61: 1432-1438 (2001)).

Elevated levels of cytokines (e.g. leptin) are likely to be the cause of uremia-asscociated cachexia in patients with chronic kidney disease (CKD). It was shown that administration of agouti-related peptide (AgRP), an endogenous melanocortin-4 receptor inverse agonist, ameloriated uremic cachexia in mice with CKD. Gains in total body weight and lean body mass were observed along with increased food intake and lower basal metabolic rate.

Furthermore, uremic cachexia in mice having a genetic MC4-R knockout was attenuated (Cheung W. et al. Role of leptin and melanocortin signaling in uremia associated cachexia. J. Clin. Invest. (2005) 115: 1659-1665). Intraperitoneal administration of small molecule MC4-R antagonist NBI-12i to uremic mice resulted in similar findings (Cheung W. et al. Peripheral administration of the melanocortin-4 receptor antagonist NBI-12i ameloriates uremia-associated cachexia in mice. J. Am. Soc. Nephrol. (2007) 18: 2517-2524).

Rats with chronic heart failure (CHF) show an impaired ability to accumulate and maintain fat mass and lean body mass. Treatment of aortic banding induced CHF in rats with AgRP resulted in significantly increases in weight gain, lean body mass, fat accumulation, kidney weights and liver weights. (Batra A.K. et al. Central melanocortin blockage attenuates cardiac cachexia in a rat model of chronic heart failure. American Federation for Medical Research, 2008 Western Regional Meeting, abstract 379).

Radiation therapy in cancer patients has been associated with anorexia and nausea (Van Cutsem E., Arends J. The causes and consequences of cancer-associated malnutrition. Eur. J. Oncol. Nurs. (2005) 9 (Suppl 2): 51-63). In a model of radiation induced anorexia in mice, AgRP treated mice ate significantly more food than animals which underwent whole body radiation (RAD) and were vehicle treated. They showed a significantly reduced loss in weight compared to RAD mice treated with vehicle (Joppa M.A. et al. Central infusion of the melanocortin receptor antagonist agouti-related peptide (AgRP(83-132)) prevents cachexia-related symptoms induced by radiation and colon-26 tumors in mice. Peptides (2007) 28: 636-642)

Clinical observations indicate that progression of amytrophic lateral sclerosis (ALS) might be inversely correlated with body weight (e.g. Ludolph AC, Neuromuscul Disord. (2006) 16 (8): 530-8). Accordingly, MC-4R inhibitors could be used to treat ALS patients.

Experimental evidence in rats suggests the involvement of central MC4-R in the mechanism of development of tolerance and dependence following chronic morphine administration. Co-administration of the melanocortin-4 receptor antagonist HS014 during chronic morphine treatment delayed the developement of tolerance and prevented withdrawal hyperalgesia (Annasaheb S.K. et al. Central administration of selective melanocortin 4 receptor antagonist HS014 prevents morphine tolerance and withdrawal hyperalgesia. Brain Research (2007) 1181: 10-20).

Modulators of the melanocortin receptor are already known from the literature. WO 2004/024720 A1 describes piperazine urea derivatives which are selective agonists of the human melanocortin-4 receptor and as such they are claimed to be useful in the treatment or prevention of obesity-related disorders.

WO 2005/047253 A1 describes 4,4-disubstituted piperidine derivatives which are postulated to function as melanocortin receptor agonists.

WO 2007/115798 A1 describes phenylpiperdine derivatives which are modulators of the human melanocortin-4 receptor. The antagonists are claimed to be useful in the treatment or prophylaxis of cancer cachexia, muscle wasting, anorexia, anxiety and/or depression, whereas the agonists are claimed to be useful in the treatment or prophylaxis of obesity, diabetes mellitus, male or female sexual dysfunction and erectile dysfunction.

WO 2007/096186 A1 discloses phenylpiperdine derivatives. Depending on their structure the compounds are agonists or antagonists of the human melanocortin-4 receptor. The antagonists are claimed to be useful in the treatment or prophylaxis of cancer cachexia, muscle wasting, anorexia, anxiety and/or depression. The agonists are claimed to be useful in the treatment or prophylaxis of obesity, diabetes mellitus, male or female sexual dysfunction and erectile dysfunction.

Substituted piperidine derivatives are also described in DE 103 00973 which relates to carboxylic acids and esters having a piperidine ring or a piperazine ring as the central core of the molecule and wherein the core is further substituted in the para-position by a 5-7-membered heterocycle, a phenyl ring, a pyridine ring or a thiazole ring. Said rings are optionally substituted by an ester group. The compounds are used in the preparation of a medicament for the treatment of headaches, non-insulin dependent diabetes mellitus (NIDDM), cardiovascularic diseases, morphintolerance, diseases of the skin, inflammations, allergic rhinitis, asthma, diseases with vascular dilatation and, consequently, with reduced blood circulation in tissues, acute or preemptive treatment of menopausal hot flashes of women with an estrogen deficiency or for the treatment of pain.

In view of the unresolved deficiencies in treatment of various diseases and disorders as discussed above, it is an object of the present invention to provide novel substituted heteroarylpiperidine derivatives with improved ability to cross the blood brain barrier, which are useful as melanocortin-4 receptor modulators to treat cachexia induced by e.g. cancer, chronic kidney disease (CKD) or chronic heart failure (CHF), muscle wasting, anorexia induced by e.g. chemotherapy or radiotherapy, anorexia nervosa, amyotrophic lateral sclerosis (ALS), pain, neuropathic pain, anxiety and depression, nausea and emesis and other diseases with MC-4R involvement.

### Summary of the Invention

The present invention relates to substituted heteroarylpiperidine derivatives of structural formula (I) 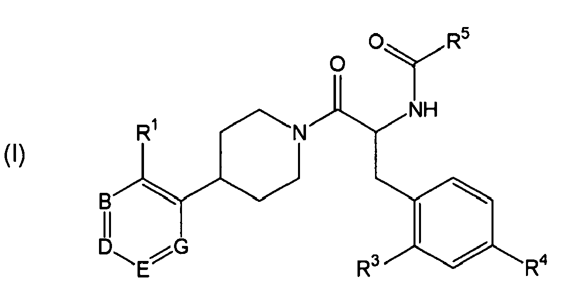 wherein R¹, R³, R⁴, R⁵, B, D, E and G are defined as described below.

The heteroarylpiperidine derivatives of structural formula (I) are effective as melanocortin receptor modulators and are particularly effective as selective melanocortin-4 receptor (MC-4R) antagonists. They are therefore useful for the treatment of disorders where the inactivation of the MC-4R is involved. The antagonists are useful for the treatment of disorders and diseases such as cachexia induced by e.g. cancer, chronic kidney disease (CKD) or chronic heart failure (CHF), muscle wasting, anorexia induced by e.g. chemotherapy or radiotherapy, anorexia nervosa, amyotrophic lateral sclerosis (ALS), pain, neuropathic pain, anxiety and depression, nausea and emesis.

Thus, the present inventions relates to compounds of formula (I) for the treatment and/or prophylaxis of cachexia induced by e.g. cancer, chronic kidney disease (CKD) or chronic heart failure (CHF), muscle wasting, anorexia induced by e.g. chemotherapy or radiotherapy, anorexia nervosa, amyotrophic lateral sclerosis (ALS), pain, neuropathic pain, anxiety and depression, nausea and emesis.

In a further aspect, the invention relates to the use of a compound of formula (I) for the preparation of a medicament for the treatment and/or prophylaxis of cachexia induced by e.g. cancer, chronic kidney disease (CKD) or chronic heart failure (CHF), muscle wasting, anorexia induced by e.g. chemotherapy or radiotherapy, anorexia nervosa, amyotrophic lateral sclerosis (ALS), pain, neuropathic pain, anxiety and depression, nausea and emesis.

The present invention also relates to pharmaceutical compositions comprising the compounds of the present invention and a pharmaceutically acceptable carrier.

### Detailed Description of the Invention

The present invention relates to substituted heteroarylpiperidine derivatives useful as melanocortin receptor modulators, in particular, selective MC-4R antagonists.

The compounds of the present invention are represented by structural formula (I) 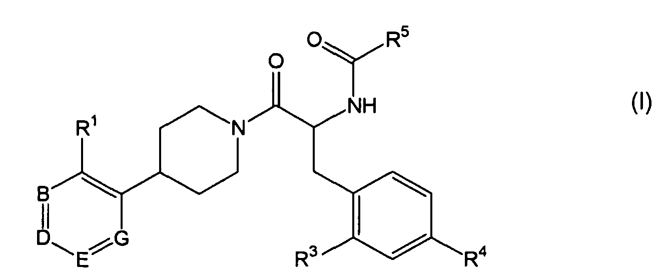 and the enantiomers, diastereomers, tautomers, solvates and pharmaceutically acceptable salts thereof,
wherein
- R¹: is -N(R¹⁰)-(C(R⁶)₂)ₘ-T
-(C(R⁶)₂)ₗ-T, or
-O-(C(R⁶)₂)ₘ-T;
- R⁶: is independently selected from
H,
F,
OH,
OCH₃,
C₁₋₆-alkyl, optionally substituted with 1 to 3 substituents selected from halogen, CN, OH and OCH₃, and
C₃₋₆-cycloalkyl, optionally substituted with 1 to 3 substituents selected from halogen, CN, OH and OCH₃;
- T: is NR⁷R⁸, 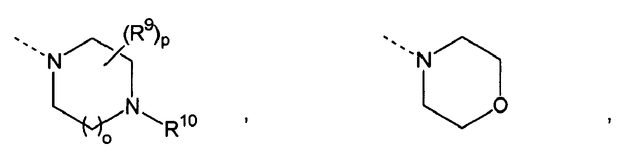 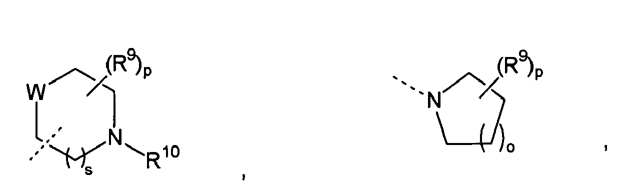 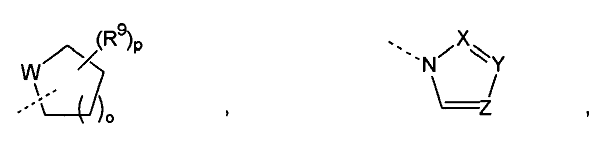 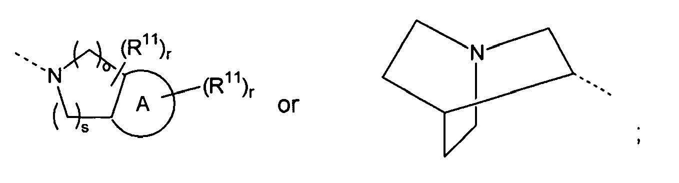
- R⁷ and R⁸: are independently from each other selected from
H,
C₁₋₆-alkyl,
C₂₋₆-alkenyl,
C₂₋₆-alkinyl, and
C₂₋₆-alkylene-O-C₁₋₆-alkyl ,
wherein each alkyl, alkenyl and alkinyl is optionally substituted by one or more halogen atoms, CN or OH;
- R⁹: is independently selected from
halogen,
CN,
OH,
C₁₋₆-alkyl, optionally substituted with 1 to 3 substituents selected from halogen, CN and OH, and
O-C₁₋₆-alkyl, optionally substituted with 1 to 3 substituents selected from halogen, CN and OH,
C₁₋₆-alkylene-O-C₁₋₆-alkyl, optionally substituted with 1 to 3 substituents selected from halogen, CN and OH, or
NR¹²R¹³;
- R¹⁰: is H, or
C₁-₆-alkyl;
- R¹¹: is independently selected from
halogen,
CN,
OH,
C₁₋₆-alkyl, optionally substituted with 1 to 3 substituents selected from halogen, CN and OH,
C₂₋₆-alkenyl,
C₂₋₆-alkinyl,
O-C₁₋₆-alkyl, optionally substituted with 1 to 3 substituents selected from halogen, CN and OH,
C₁₋₆-alkylene-O-C₁₋₆-alkyl, optionally substituted with 1 to 3 substituents selected from halogen, CN and OH,
C₀₋₆-alkyl-C₃₋₆-cycloalkyl,
-OC(O)C₁₋₆-alkyl,
-NH₂,
-NH(C₁₋₆-alkyl), and
-N(C₁₋₆-alkyl)₂;
- R¹² and R¹³: are independently from each other selected from
C₁₋₆-alkyl, optionally substituted with OH,
C₂₋₆-alkenyl,
C₂₋₆-alkinyl,
C₂₋₆-alkylene-O-C₁₋₆-alkyl, and
C₂₋₆-alkylene-N-(C₁₋₆-alkyl)₂;
- W: is CH, O or NR¹⁰;
- X: is CH or N;
- Y: is CH or N;
- Z: is CH or N;
- A: is a 3-7-membered saturated, unsaturated or aromatic ring containing 0-2 nitrogen atoms;
- B: is CR² or N;
- G: is CR² or N;
- D: is CR² or N;
- E: is CR² or N;
with the proviso that one or two of the variables B, G, D and E must be N;
- R²: is independently selected from
H,
F,
Cl,
CH₃,
- R³: is OCH₃, and
CF₃; H,
Cl,
F, or
CH₃;
- R⁴: is Cl,
F or
CH₃,
- R⁵: is -O-(CH₂)ₜ-Q or -O-R¹⁵;
- Q: is a 3-6-membered, saturated or partially unsaturated carbocycle or heterocycle containing in the ring a nitrogen or an oxygen atom, wherein the carbocycle or hetercycle is optionally substituted by 1 to 4 same or different substitutents R¹⁴
- R¹⁴: is selected from
halogen,
CN,
OH,
C₁₋₆-alkyl, optionally substituted with 1 to 3 substituents selected from halogen, CN and OH,
C₂₋₆-alkenyl,
C₂₋₆-alkinyl,
O-C₁₋₆-alkyl, optionally substituted with 1 to 3 substituents selected from halogen, CN and OH,
C₁₋₆-alkylene-O-C₁₋₆-alkyl, optionally substituted with 1 to 3 substituents selected from halogen, CN and OH,
C₀₋₆-alkyl-C₃₋₆-cycloalkyl,
-OC(O)C₁₋₆-alkyl,
-C₀₋₆-NH_{2,}
-C₀₋₆-NH(C₁₋₆-alkyl), and
-C₀₋₆-N(C₁₋₆-alkyl)₂;
- R¹⁵: is selected from
C₁₋₆ alkyl, optionally substituted with OH or halogen,
C₁₋₆-alkylene-O-C₁₋₆-alkyl, and
C₁₋₆-alkylene-N(C₁₋₆-alkyl)₂;
- I: is 0, 1, 2, 3, or 4;
- m: is 0, 1, 2, 3, or 4;
- o: is 0, 1, or 2;
- p: is 0, 1, 2, 3, or 4;
- r: is 0, 1, 2, 3, or 4;
- s: is 1, or 2; and
- t: is 0, 1 or 2.

In another preferred embodiment in combination with any of the above or below embodiments, the compounds according to formula (I) adopt the structural conformation of the following stereoisomer formula (I'): 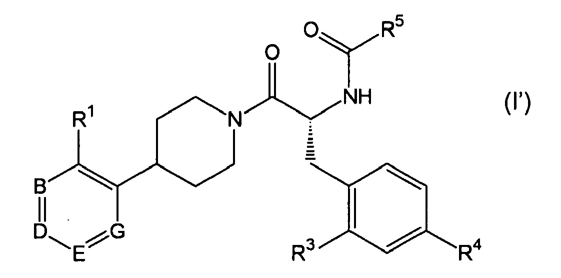 wherein B, G, D, E, R¹, R³, R⁴ and R⁵ are as defined above.

In another preferred embodiment in combination with any of the above or below embodiments, the moiety 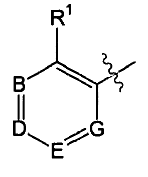 in general formula (I) is selected from the following structures: 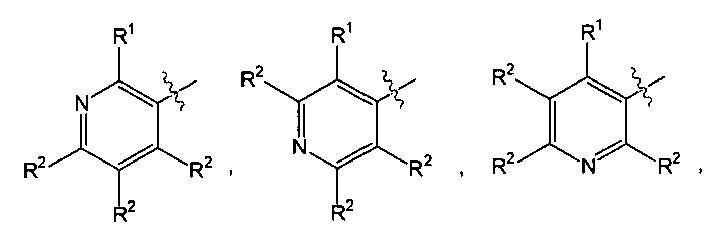 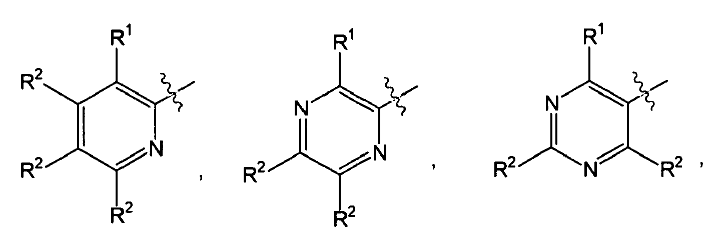 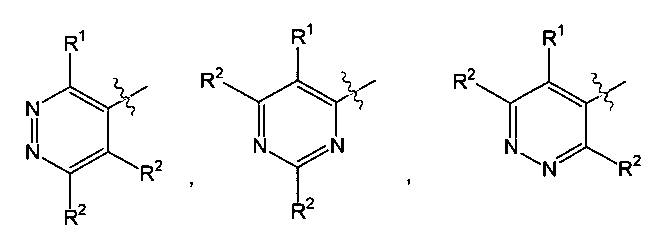 and

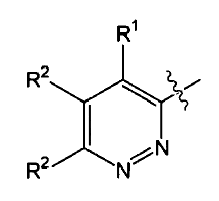

Therein, the substituent R² is defined as above. In another preferred embodiment in combination with any of the above or below embodiments, R² represents H, Cl, F or CH₃, more preferably, R² represents H or CH₃.

In another preferred embodiment in combination with any of the above or below embodiments, the moiety 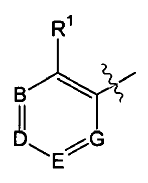 represents the following structures:

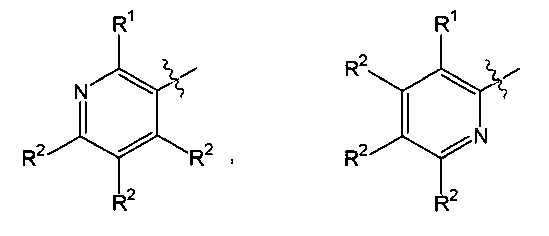

In another preferred embodiment in combination with any of the above or below embodiments, the substituent R¹ represents -N(R¹⁰)-(C(R⁶)₂)ₘ-T, -(C(R⁶)₂)ₗ-T, or -O-(C(R⁶)₂)ₘ-T, wherein R⁶ and R¹⁰ are preferably independently selected from the group consisting of H and C₁₋₆-alkyl.

In another preferred embodiment in combination with any of the above or below embodiments, R³ represents H, Cl, or CH₃, more preferably Cl. In an alternative preferred embodiment, R³ represents F.

In another preferred embodiment in combination with any of the above or below embodiments, R⁴ represents Cl.

In another preferred embodiment in combination with any of the above or below embodiments, R⁵ is -O-(CH₂)ₜ-Q.

In another preferred embodiment in combination with any of the above or below embodiments, R⁵ is -O-R¹⁵.

In another preferred embodiment in combination with any of the above or below embodiments, the group Q is a 3-6-membered, saturated or partially unsaturated carbocycle or heterocycle containing in the ring a nitrogen or an oxygen atom, wherein the carbocycle or hetercycle is optionally substituted by 1 to 3 same or different substitutents R¹⁴, preferably wherein the carbocycle or hetercycle is optionally substituted by 1 or 2 same or different substitutents R¹⁴.

In another preferred embodiment in combination with any of the above or below embodiments, the group Q is a 3-6-membered, saturated carbocycle or heterocycle containing in the ring a nitrogen or an oxygen atom, wherein the carbocycle or hetercycle is optionally substituted by 1 to 4 same or different substitutents R¹⁴, preferably wherein the carbocycle or hetercycle is optionally substituted by 1 to 3 same or different substitutents R¹⁴, more preferably wherein the carbocycle or hetercycle is optionally substituted by 1 or 2 same or different substitutents R¹⁴.

In another preferred embodiment in combination with any of the above or below embodiments, at least one of R⁷ and R⁸ is selected from the group consisting of C₁₋₆-alkyl, C₂₋₆-alkenyl, C₂₋₆-alkinyl and C₂₋₆-alkylene-O-C₁₋₆-alkyl, more preferably from C₂₋₆-alkenyl, C₂₋₆-alkinyl and C₂₋₆-alkylene-O-C₁₋₆-alkyl.

In another preferred embodiment in combination with any of the above or below embodiments, R⁹ is independently selected from the group consisting of halogen, CN, OH, C₁₋₆-alkyl optionally substituted with 1 to 3 substituents selected from halogen, CN and OH, and O-C₁₋₆-alkyl optionally substituted with 1 to 3 substituents selected from halogen, CN and OH.

In another preferred embodiment in combination with any of the above or below embodiments, R¹⁴ is selected from
halogen,
OH,
C₁₋₄-alkyl, optionally substituted with 1 or 2 substituents selected from halogen and OH,
C₂₋₄-alkenyl,
C₂₋₄-alkinyl,
O-C₁₋₄-alkyl, optionally substituted with 1 or 2 substituents selected from halogen and OH,
C₁₋₄-alkylene-O-C₁₋₄-alkyl, optionally substituted with 1 or 2 substituents selected from halogen and OH,
C₀₋₄-alkyl-C₃₋₆-cycloalkyl,
-C₀₋₄-NH₂,
-CO-₄-NH(C₁₋₄-alkyl), and
-C₀₋₄-N(C₁₋₄-alkyl)₂;

In another preferred embodiment in combination with any of the above or below embodiments, R¹⁵ is selected from
C₁₋₆-alkyl, optionally substituted with OH or halogen,
C₁₋₆-alkylene-O-C₁₋₄-alkyl, and
C₁₋₆-alkylene-N(C₁₋₄-alkyl)₂.

In another preferred embodiment in combination with any of the above or below embodiments, the variable I is preferably selected from 2 or 3.

In another preferred embodiment in combination with any of the above or below embodiments, the variable m is preferably selected from 2, 3 or 4, more preferably from 2 or 3.

In another preferred embodiment in combination with any of the above or below embodiments, as regards compounds of formula (I), T is preferably selected from the group consisting of the following radicals:

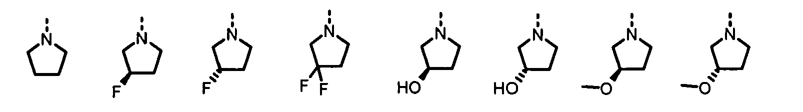

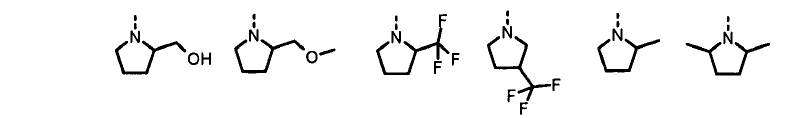

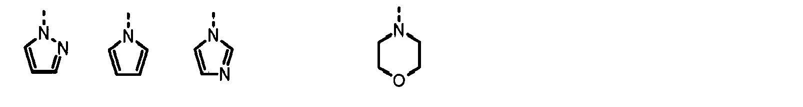

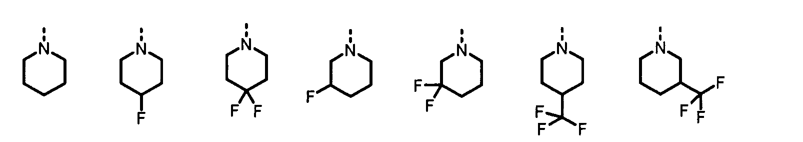

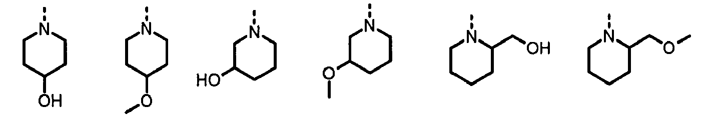

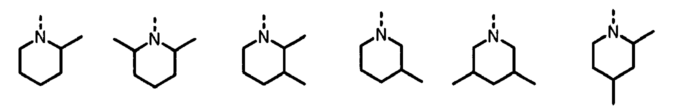

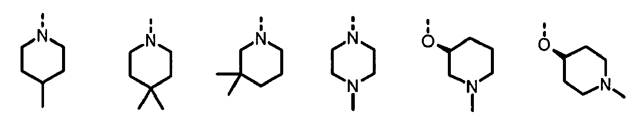

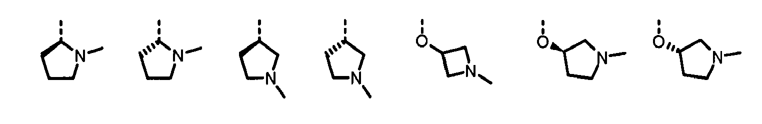

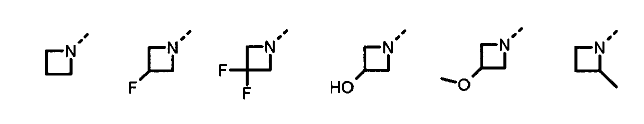

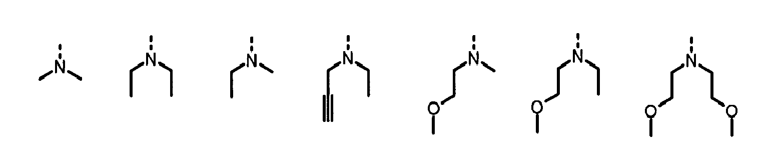

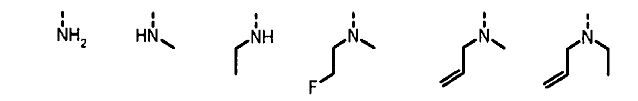

In another preferred embodiment in combination with any of the above or below embodiments, R⁵ is preferably selected from the group consisting of

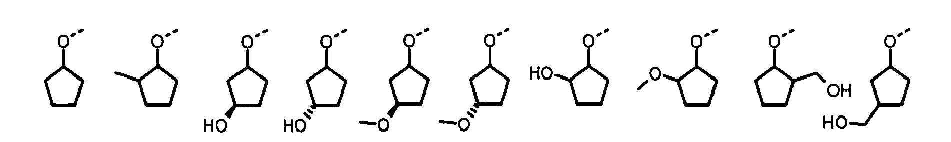

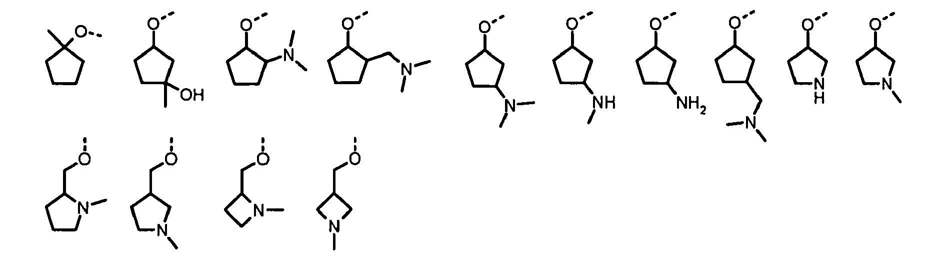

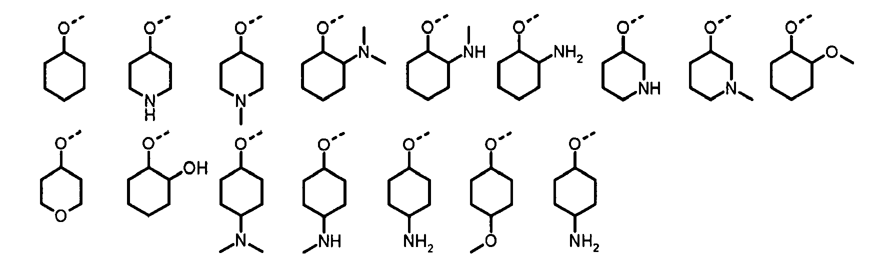

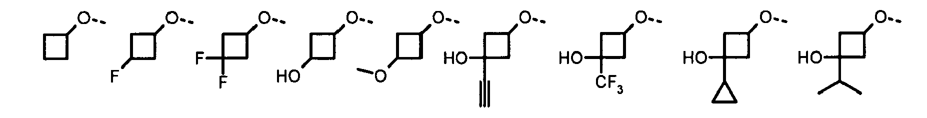

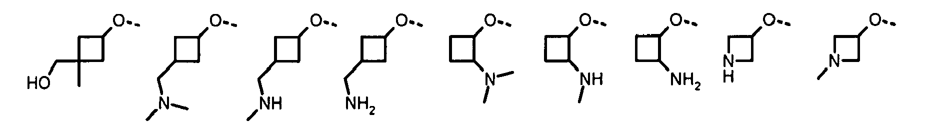

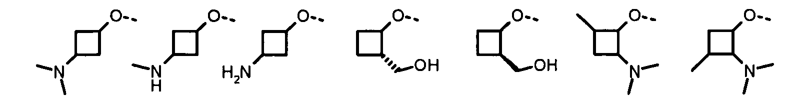

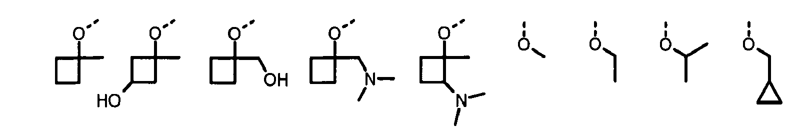

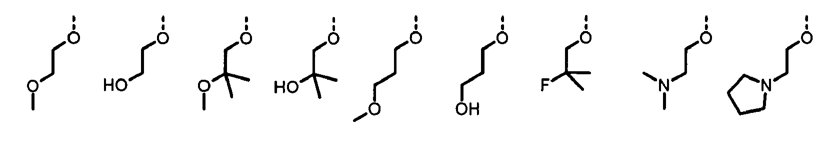

Compounds of the formula (I) in which some or all of the above-mentioned groups have the preferred or more preferred meanings are also an object of the present invention.

In the above and the following, the employed terms have the meaning as described below:
Alkyl is a straight chain or branched alkyl having 1 to 6 carbon atoms, such as methyl, ethyl, n-propyl, isopropyl, n-butyl, isobutyl, tert-butyl, n-pentyl, isopentyl, neopentyl, or hexyl.

Alkenyl is a straight chain or branched alkyl having 2 to 6 carbon atoms and which contains at least one carbon-carbon double bond, such as vinyl, allyl, 1-propenyl, 2-butenyl, 2-methyl-2-butenyl, isopropenyl, pentenyl, or hexenyl.

Alkinyl is a straight chain or branched alkyl having 2 to 6 carbon atoms and which contains at least one carbon-carbon triple bond, such as ethinyl, 1-propinyl, 1-butinyl, 2-butinyl, pentinyl or hexinyl.

A 3-7-membered, saturated, unsaturated or aromatic ring containing 0-2 nitrogen atoms encompasses a 3-7-membered saturated carbocycle such as cyclopropyl, cyclobutyl, cyclopentyl, cyclohexyl or cycloheptyl. Said term further encompasses 3-7-membered unsaturated carbocycles such as cyclobutenyl, cyclopentenyl, cyclohexenyl, cycloheptenyl, cyclohexa-1,4-diene or cycloheptadienes, or aromatic rings such as benzene. Nitrogen-containing, 3-7-membered, saturated, unsaturated or aromatic heterocycles are further encompassed by the above term. Examples thereof include azetidine, pyrrolidine, piperidine, azepane, piperazine, pyridine, pyrimidine, pyrazine, pyrrole, imidazole, and pyrazole.

The compounds of structural formula (I) are effective as melanocortin receptor modulators and are particularly effective as selective antagonists of MC-4R. They are therefore useful for the treatment and/or prevention of disorders responsive to the inactivation of MC-4R, such as cachexia induced by e.g. cancer, chronic kidney disease (CKD) or chronic heart failure (CHF), muscle wasting, anorexia induced by e.g. chemotherapy or radiotherapy, anorexia nervosa, amyotrophic lateral sclerosis (ALS), pain, neuropathic pain, anxiety and depression, nausea and emesis and other diseases with MC-4R involvement.

In another preferred embodiment in combination with any of the above or below embodiments, the compounds of structural formula (I) are useful for the treatment and/or prevention of cachexia.

In another preferred embodiment in combination with any of the above or below embodiments, the compounds of structural formula (I) are useful for the treatment and/or prevention of cachexia, wherein cachexia is selected from cancer cachexia, cachexia induced by chronic kidney disease (CKD) or cachexia induced by chronic heart failure (CHF).

In another preferred embodiment in combination with any of the above or below embodiments, the compounds of structural formula (I) are useful for the prophylaxis or treatment of muscle wasting.

In another preferred embodiment in combination with any of the above or below embodiments, the compounds of structural formula (I) are useful for the prophylaxis or treatment of anorexia.

In another preferred embodiment in combination with any of the above or below embodiments, the compounds of structural formula (I) are useful for the prophylaxis or treatment of anorexia, wherein the anorexia is selected from anorexia nervosa or anorexia induced by radiotherapy or chemotherapy.

In another preferred embodiment in combination with any of the above or below embodiments, the compounds of structural formula (I) are useful for the prophylaxis or treatment of anxiety and/or depression.

In another preferred embodiment in combination with any of the above or below embodiments, the compounds of structural formula (I) are useful for the prophylaxis or treatment of amyotrophic lateral sclerosis (ALS).

In another preferred embodiment in combination with any of the above or below embodiments, the compounds of structural formula (I) are useful for the prophylaxis or treatment of pain, in particular neuropathic pain.

In another preferred embodiment in combination with any of the above or below embodiments, the compounds of structural formula (I) are useful for the prophylaxis or treatment of nausea and emesis.

### Optical Isomers - Diastereomers - Geometric Isomers - Tautomers

Compounds of structural formula (I) contain one or more asymmetric centers and can occur as racemates and racemic mixtures, single enantiomers, diastereomeric mixtures and individual diastereomers. The present invention is meant to comprehend all such isomeric forms of the compounds of structural formula (I).

Compounds of structural formula (I) may be separated into their individual diastereoisomers by, for example, fractional crystallization from a suitable solvent, for example methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration.

Alternatively, any stereoisomer of a compound of the general formula (I) may be obtained by stereospecific synthesis using optically pure starting materials or reagents of known absolute configuration.

### Salts

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, furnaric, gluconic, glutamic, hydrobromic, hydrochloric, isethionic, lactic, maleic, malic, mandelic, methanesulfonic, malonic, mucic, nitric, parnoic, pantothenic, phosphoric, propionic, succinic, sulfuric, tartaric, p-toluenesulfonic, trifluoroacetic acid and the like. Particularly preferred are citric, fumaric, hydrobromic, hydrochloric, maleic, phosphoric, sulfuric and tartaric acids.

It will be understood that, as used herein, references to the compounds of formula (I) are meant to also include the pharmaceutically acceptable salts.

### Utility

The compounds of formula (I) are melanocortin receptor antagonists and as such are useful in the treatment, control or prevention of diseases, disorders or conditions responsive to the inactivation of one or more of the melanocortin receptors including, but not limited to, MC-1R, MC-2R, MC-3R, MC-4R or MC-5R. Such diseases, disorders or conditions include, but are not limited to, cachexia induced by e.g. cancer, chronic kidney disease (CKD) or chronic heart failure (CHF), muscle wasting, anorexia induced by e.g. chemotherapy or radiotherapy, anorexia nervosa, amyotrophic lateral sclerosis (ALS), pain, neuropathic pain, anxiety and depression, nausea and emesis.

The compounds of formulas (I) can be further used in the treatment, control or prevention of hypertension, hyperlipidemia, osteoarthritis, cancer, gall bladder disease, sleep apnea, compulsion, neuroses, insomnia/sleep disorder, substance abuse, pain, fever, inflammation, immune-modulation, rheumatoid arthritis, skin tanning, acne and other skin disorders, neuroprotective and cognitive and memory enhancement including the treatment of Alzheimer's disease.

### Administration and Dose Ranges

Any suitable route of administration may be employed for providing a mammal, especially a human with an effective dosage of a compound of the present invention. For example, oral, rectal, topical, parenteral, ocular, pulmonary, nasal and the like may be employed. Dosage forms include tablets, troches, dispersions, suspensions, solutions, capsules, creams, ointments, aerosols and the like. Preferably compounds of formula (I) are administered orally or topically.

The effective dosage of active ingredient employed may vary depending on the particular compound employed, the mode of administration, the condition being treated and the severity of the condition being treated. Such dosage may be ascertained readily by a person skilled in the art.

When treating cachexia induced by e.g. cancer, chronic kidney disease (CKD) or chronic heart failure (CHF), muscle wasting, anorexia induced by e.g. chemotherapy or radiotherapy, anorexia nervosa, amyotrophic lateral sclerosis (ALS), pain, neuropathic pain, anxiety and depression, nausea and emesis generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

### Formulation

The compounds of formula (I) are preferably formulated into a dosage form prior to administration. Accordingly the present invention also includes a pharmaceutical composition comprising a compound of formula (I) and a suitable pharmaceutical carrier.

The present pharmaceutical compositions are prepared by known procedures using well-known and readily available ingredients. In making the formulations of the present invention, the active ingredient (a compound of formula (I)) is usually mixed with a carrier, or diluted by a carrier, or enclosed within a carrier, which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient.

### Preparation of Compounds of the Invention

When describing the preparation of the present compounds of formula (I), the terms "A moiety", "B moiety" and "C moiety" are used below. This moiety concept is illustrated below:

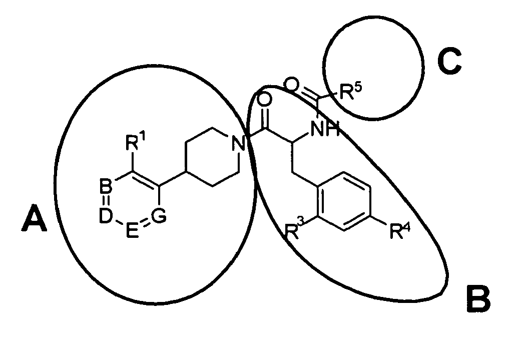

The preparation of the compounds of the present invention may be carried out via sequential or convergent synthetic routes. The skilled artisan will recognize that, in general, the A and B moieties of a compound of formula (I) are connected via amide bonds. The skilled artist can, therefore, readily envision numerous routes and methods of connecting the two moieties via standard peptide coupling reaction conditions.

The phrase "standard peptide coupling reaction conditions" means coupling a carboxylic acid with an amine using an acid activating agent such as EDCI, dicyclohexylcarbodiimide and benzotriazol-1-yloxytris(dimethylamino)-phosphonium hexafluorophosphate, in a inert solvent such as DCM, in the presence of a catalyst such as HOBt. The uses of protective groups for amine and carboxylic acids to facilitate the desired reaction and minimize undesired reactions are well documented. Conditions required to remove protecting groups which may be present can be found in Greene et al., Protective Groups in Organic Synthesis, John Wiley & Sons, Inc., New York, NY 1991.

Protecting groups like Z, Boc and Fmoc are used extensively in the synthesis, and their removal conditions are well known to those skilled in the art. For example, removal of Z groups can he achieved by catalytic hydrogenation with hydrogen in the presence of a noble metal or its oxide, such as palladium on activated carbon in a protic solvent, such as ethanol. In cases where catalytic hydrogenation is contraindicated by the presence of other potentially reactive functionality, removal of Z can also be achieved by treatment with a solution of hydrogen bromide in acetic acid, or by treatment with a mixture of TFA and dimethylsulfide. Removal of Boc protecting groups is carried out in a solvent such as methylene chloride, methanol or ethyl acetate with a strong acid, such as TFA or HCl or hydrogen chloride gas.

The B and C moieties of a compound of formula (I) are linked together via a carbamate function. The skilled artist can, therefore, readily envision numerous routes and methods of connecting the two moieties using different well known methods.

The compounds of formula (I), when existing as a diastereomeric mixture, may be separated into diastereomeric pairs of enantiomers by fractional crystallization from a suitable solvent such as methanol, ethyl acetate or a mixture thereof. The pair of enantiomers thus obtained may be separated into individual stereoisomers by conventional means by using an optically active acid as a resolving agent. Alternatively, any enantiomer of a compound of the formula (I) may be obtained by stereospecific synthesis using optically pure starting materials or reagents of known configuration.

The compounds of formula (I) of the present invention can be prepared according to the procedures of the following schemes and examples, using appropriate materials and are further exemplified by the following specific examples. Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described previously. The free amine bases corresponding to the isolated salts can be generated by neutralization with a suitable base, such as aqueous sodium hydrogencarbonate, sodium carbonate, sodium hydroxide and potassium hydroxide, and extraction of the liberated amine free base into an organic solvent followed by evaporation. The amine free base isolated in this manner can be further converted into another pharmaceutically acceptable salt by dissolution in an organic solvent followed by addition of the appropriate acid and subsequent evaporation, precipitation or crystallization. All temperatures are degrees Celsius.

In the schemes, preparations and examples below, various reagent symbols and abbreviations have the following meanings:
- Ac₂O: acetic anhydride
- AcOH: acetic acid
- Boc: tert-butoxycarbonyl
- Bu: butyl
- BuLi: butyllithium
- DBU: 1,8-diazabicyclo[5.4.0]undec-7-ene
- DCC: N,N'-dicyclohexylcarbodiimide
- DCE: 1,2-dichloroethane
- DCM: dichloromethane
- DEAD: diethyl azodicarboxylate
- DIAD: diisopropyl azodicarboxylate
- DIC: N,N'-diisopropylcarbodiimide
- DIEA: ethyl-diisopropylamine
- DMA: N,N-dimethylacetamide
- DMAP: 4-dimethylaminopyridine
- DMF: N,N-dimethylformamide
- DMS: dimethylsulfide
- DMSO: dimethylsulfoxide
- dppf: 1,1'-bis(dipheny)phosphino)-ferrocen
- EDCI: 1-(3-dimethylaminopropyl)-3-ethylcarbodiimide hydrochloride
- Et₂O: diethyl ether
- EtOAc: ethyl acetate
- EtOH: ethanol
- Fmoc: 9-fluorenylmethyloxycarbonyl
- h: hour(s)
- HOBt: 1-hydroxybenzotriazole hydrate
- HTMP: 2,2,6,6-tetramethylpiperidine
- MeCN: acetonitrile
- MeOH: methanol
- NMM: N-methylmorpholine
- mp.: melting point
- min: minute(s)
- MW: molecular weight
- PG: protecting group
- PPh₃: triphenylphosphine
- RT: room temperature
- TEA: triethylamine
- TFA: trifluoroacetic acid
- THF: tetrahydrofurane
- t_{R} (min): HPLC retention time
- Z: benzyloxycarbonyl
- Z-OSu: N-(benzyloxycarbonyloxy)succinimide

2-Fluoro-3-iodo-pyrazine was prepared as described in Tetrahedron 1998, 54, 4899-4912. 4-Fluoro-3-iodo-pyridine was prepared as described in Tetrahedron 1993, 49, 49-64.

### Reaction scheme 1:

### Directed ortho-metallation

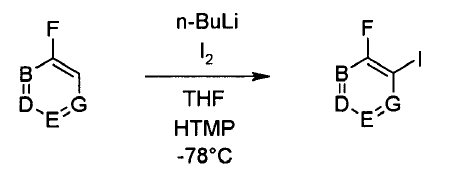

The starting material for the synthesis of heteroarylpiperidines, ortho-fluoro-iodopyridines, -pyridazines and -pyrazines, can be prepared as shown in Reaction scheme 1. A fluoro-substituted heteroaryl can be metallated with an amide prepared from a reagent such as n-butyllithium and an amine such as diisopropylamine or 2,2,6,6-tetramethylpiperidine at an appropriate temperature in a suitable solvent such as THF. The resulting lithio derivatives can subsequently be reacted with iodine to yield the desired compounds.

### Reaction scheme 2:

### Acetylation

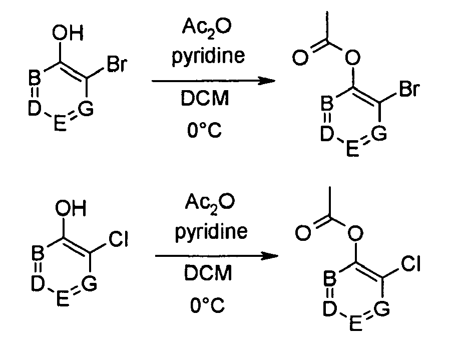

Another starting material for the synthesis of heteroarylpiperidines, ortho-acetoxysubstituted bromo- or chloropyridines, -pyridazines and -pyrazines, can be prepared as shown in Reaction scheme 2. A bromo- or chloroheteroaryl containing a hydroxy group in ortho-position to the halogen atom can be reacted with an acetylating reagent such as acetic anhydride in the presence of a suitable base such as pyridine in an appropriate solvent like DCM at a suitable temperature.

### Reaktion scheme 3:

### Negishi Coupling Reaction of Bromo- or Iodosubstituted Heteroaryls

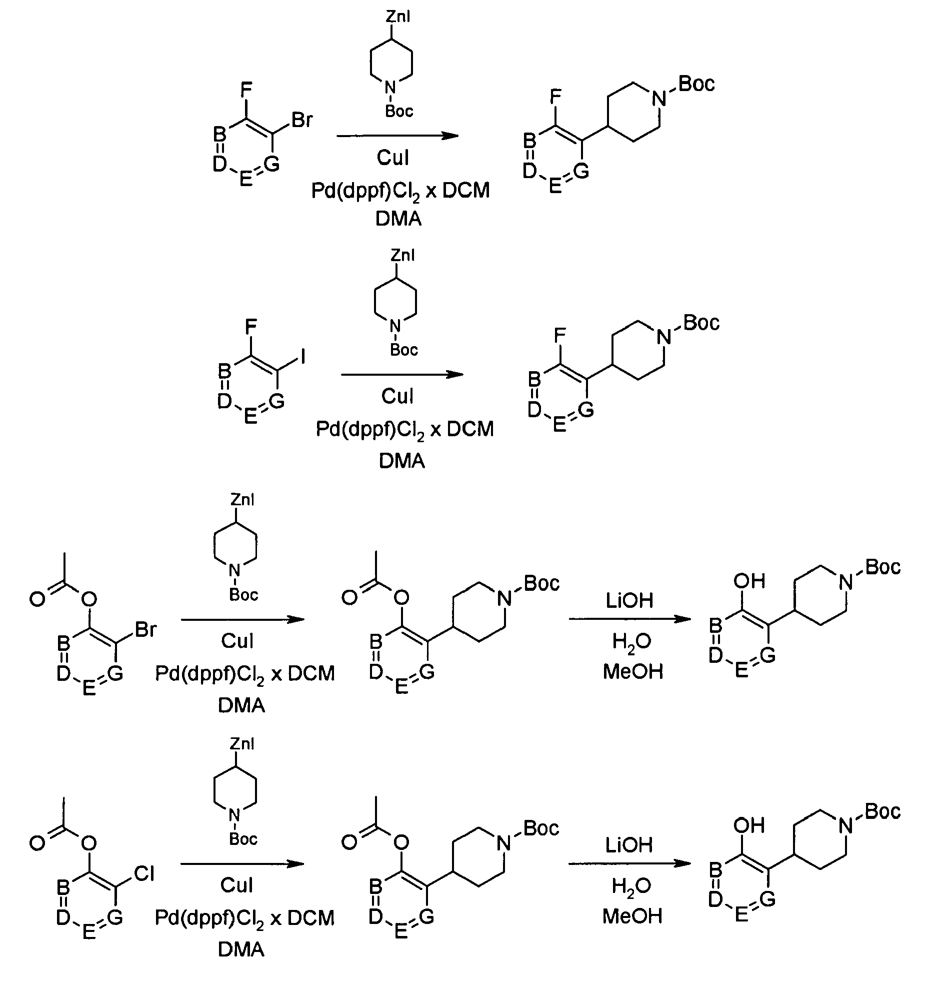

As shown in Reaction scheme 3, ortho-fluoro-bromopyridines, pyridazines and -pyrazines can be subjected to a Negishi coupling with (1-tert-butoxycarbonylpiperidin-4-yl)(iodo)zinc (J. Org. Chem. 2004, 69, 5120-5123) in the presence of copper(I) iodide and dichloro(1,1'-bis(diphenyl-phosphino)-ferrocene)palladium(II) DCM adduct in an inert solvent such as DMA to yield the resulting arylpiperidine. The same product is obtained, when ortho-fluoroiodoopyridines, -pyridazines and -pyrazines are used as starting material.

The Negishi coupling can alternatively be performed using the ortho-acetoxysubstituted bromopyridines, -pyridazines and -pyrazines from Reaction scheme 2 as starting material. The free alcohol is obtained by saponification of the acetic acid ester with a base such as lithium hydroxide in a suitable solvent such as mixture of water and methanol. Usage of ortho-acetoxy-chloropyridines, -pyridazines and -pyrazines as starting materials results in formation of the same products.

### Reaktion scheme 4:

### Negishi Coupling Reaction of Heteroaryls with a Carboxaldehyde or Benzylamine Function

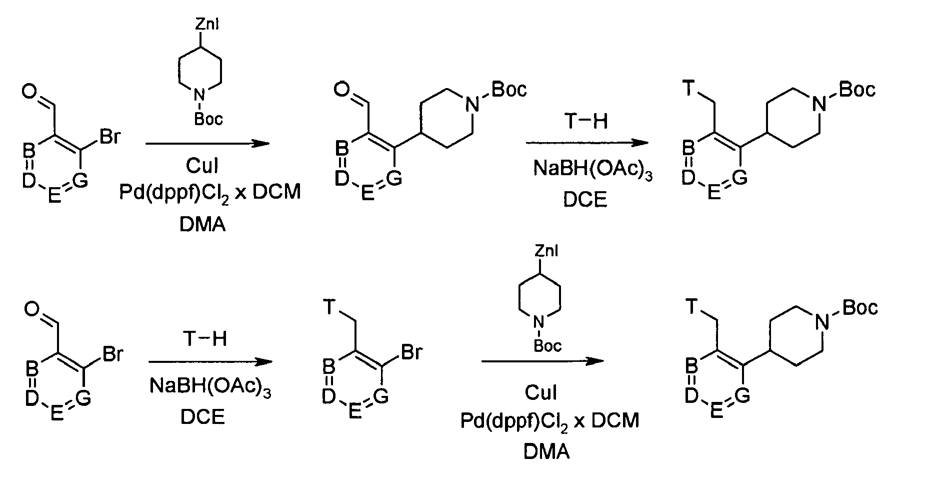

Optionally substituted ortho-carboxy-bromopyridines, pyridazines and -pyrazines can be subjected to a Negishi coupling as depicted in Reaction scheme 4. Reaction with (1-tert-butoxycarbonylpiperidin-4-yl)(iodo)zinc in the presence of copper(I) iodide and dichloro(1,1'-bis(diphenyl-phosphino)-ferrocene)palladium(II) DCM adduct in an inert solvent such as DMA leads to the resulting arylpiperidine. Reductive amination with a capping group T-H in the presence of a reducing agent such as sodium triacetoxyborohydride in a suitable solvent such as 1,2-dichloroethane leads to the Boc-protected A-moiety.

Alternatively, optionally substituted ortho-carboxy-bromopyridines, pyridazines and -pyrazines can first be subjected to a reductive amination step before the Negishi coupling is performed.

The reaction sequences described above can also be performed using optionally substituted ortho-carboxy-chloropyridines, pyridazines and -pyrazines.

### Reaktion scheme 5:

### Aminoalcohol Preparation

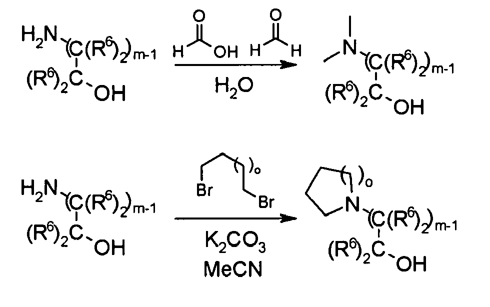

N-substituted amino alcohols HO(C(R⁶)₂)ₘ-T can be obtained as described in Reaction scheme 5. Reaction of an optionally substituted amino alcohol HO(C(R⁶)₂)ₘNH₂ with a mixture of formic acid and formaldehyde in a suitable solvent such as water at a given temperature results in formation of the corresponding N,N-dimethylated amino alcohols.

Cyclic analogs of such amino alcohols can be obtained by reacting optionally substituted amino alcohol HO(C(R⁶)₂)ₘNH₂ with α,ω-dibromoalkanes in the presence of a base such as potassium carbonate in an appropriate solvent like acetonitrile.

### Reaktion scheme 6:

### Epoxides as Aminoalcohol Precursor

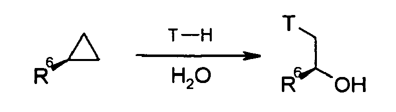

As shown in Reaction scheme 6, optionally substituted epoxides can be reacted in a regioselective way with an appropriate amine T-H in a suitable solvent like water to form α-substituted β-aminoalcohols.

### Reaktion scheme 7:

### Nucleophilic Aromatic Substitution Reaction

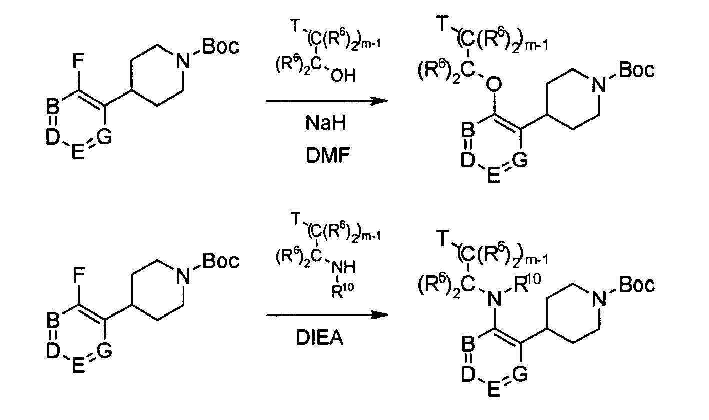

As shown in Reaction scheme 7, fluoro-substituted pyridyl-, pyridazyl- and pyrazinylpiperidines can be subjected to a nucleophilic aromatic substitution reaction with a ω-T-capped alkylalcohol in the presence of a base such as sodium hydride in a solvent such as DMF at a suitable temperature to obtain the Boc-protected A moiety.

Alternatively, the fluoro-substituted heteroarylpiperidines can also be reacted with a ω-T-capped primary or secondary alkylamine in the presence of a base like BuLi or DIEA in an appropriate solvent like THF or without a solvent at a suitable temperature to obtain the Boc-protected A moiety.

### Reaction scheme 8

### Synthesis of A Moieties Containing Cyclic Amines

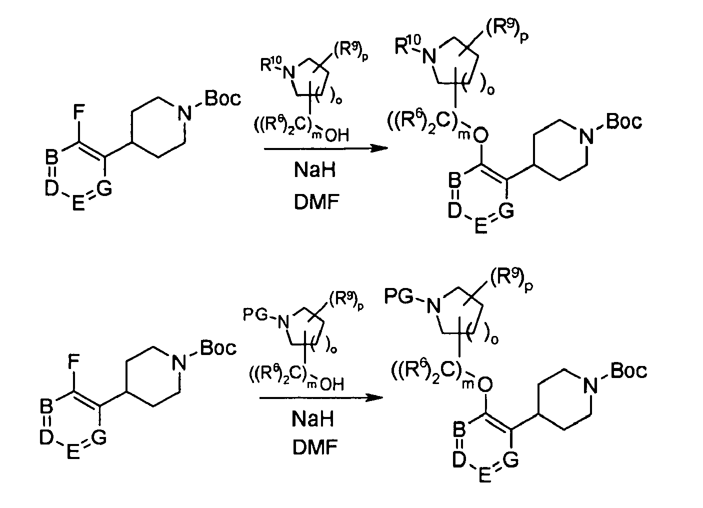

As shown in Reaction scheme 8, the intermediate product from Reaction scheme 3, optionally substituted fluoropyridines, -pyridazines and -pyrazines, can also be subjected to a nucleophilic aromatic substitution reaction with an alcohol which contains a cyclic tertiary amine moiety, in the presence of a base such as sodium hydride in a suitable solvent such as DMF to give the Boc-protected A moieties.

Similarly, an alcohol containing a protected cyclic secondary amine moiety can be introduced as building block using the conditions described above. The protecting group has to be orthogonal to the Boc-protecting group used for protection of the piperidine. After coupling of the A moiety with the B-C moiety this protecting group can be removed using standard methods.

### Reaction scheme 9:

### Alternative Synthesis of A Moieties with Alkylether Spacer (R¹ = -O(C(R⁶)₂)ₘ-T)

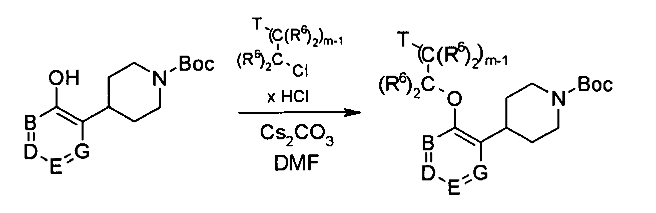

The synthesis of A Moieties bearing an alkylether spacer (R¹ = -O(C(R⁶)₂)ₘ-T) can alternatively be performed as depicted in Reaction scheme 9. The Boc-protected piperidine is reacted with an alkylchloride or alkylbromide bearing the capping group T in the presence of a base such as Cs₂CO₃ or NaH in an appropriate solvent such as DMF to give the Boc-protected A moiety.

### Reaction scheme 10:

### Synthesis of A Moieties with Alkylether Spacer (R¹ = -O(C(R⁶)₂)ₘ-T) Using Mitsunobu Conditions

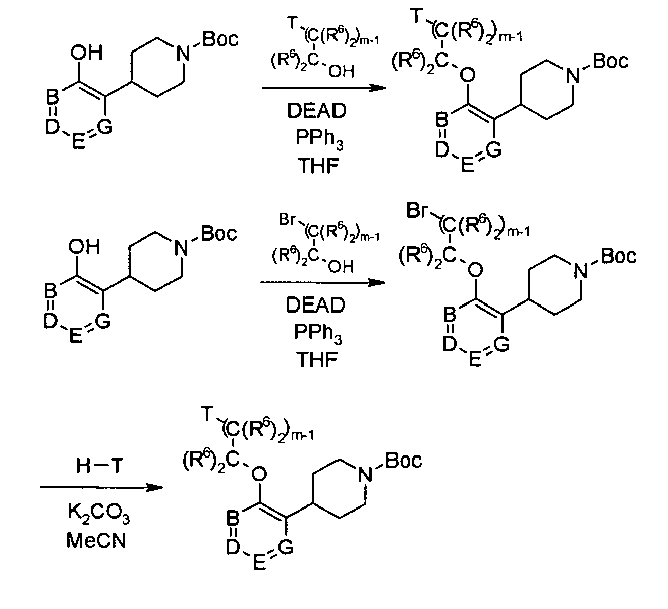

As shown in Reaction scheme 10, the intermediate product from Reaction scheme 3, optionally substituted (hydroxyheteraoaryl)piperidines, can also be alkylated with an ω-T-capped alkylalcohol in the presence of a reagent such as DEAD or DIAD and a phosphine such as PPh₃ in a suitable solvent such as THF to give the Boc-protected A moieties.

Similarly, the same intermediate can be reacted with an ω-bromo alkylalcohol, using the reaction conditions described above, to give access to the corresponding ether which subsequently can be used to alkylate the capping group T in the presence of a suitable base such as K₂CO₃ or NaH, in an appropriate solvent such as MeCN, THF, or DMF, at a suitable temperature, to yield the Boc-protected A moieties.

### Reaction scheme 11:

### Synthesis of 5-piperidin-4-yl-pyrimidine-derived A moieties

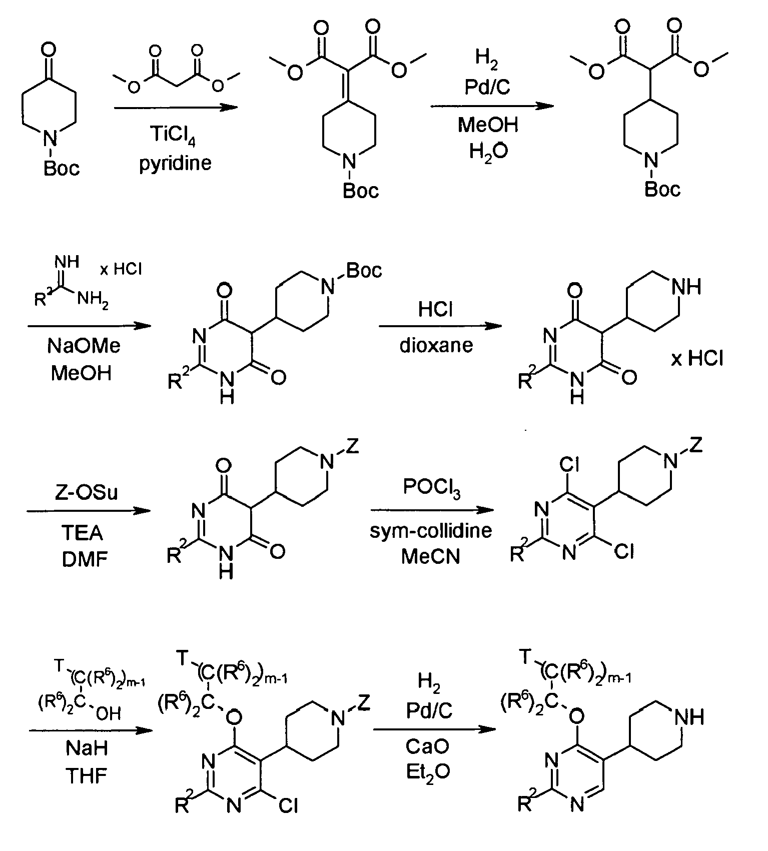

5-Piperidin-4-yl-pyrimidine-derived A moieties can be synthesized as shown in Reaction scheme 11. Boc-4-piperidone can be reacted with a malonic acid diester in the presence of a reagent such as titanium tetrachloride and a base such as pyridine at an appropriate temperature. The product of this reaction can be hydrogenated using a catalyst such as 10% palladium on charcoal in a suitable solvent such as a mixture of water and methanol to yield the corresponding Boc-2-piperidin-4-yl-malonic acid diester. Reaction with an amidine hydrochloride in the presence of a base such as sodium methoxide in a solvent like methanol leads to optionally substituted 1H-pyrimidine-4,6-dione which after exchanging the Boc protecting group by a Z protecting group can be converted to the 4,6-dichloro-pyrimidine using a reagent such as POCl₃ in the presence of a suitable base such as sym-collidine in an appropriate solvent like acetonitrile. Optionally substituted 4,6-dichloro-pyrimidine can be reacted with an ω-T-capped alkylalcohol in the presence of a base such as sodium hydride in a suitable solvent like THF. Subsequent hydrogenation using a catalyst such as 10% palladium on charcoal in the presence of calcium oxide in an appropriate solvent like diethyl ether yields the 5-piperidin-4-yl-pyrimidine-derived A moieties.

### Reaction scheme 12

### A Moiety Deprotection

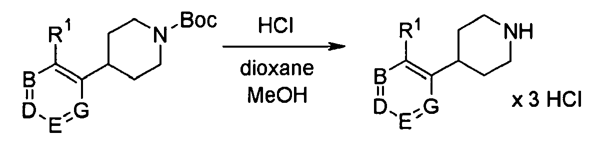

Generally, the starting material of Boc-protected heteroarylpiperidine (A moiety) can be deprotected in the presence of TFA/CH₂Cl₂, HCl/EtOAc, HCl/dioxane or HCl in MeOH/dioxane with or without a cation scavenger, such as dimethyl sulfide (DMS) before being subjected to the coupling procedure. It can be converted to the free base before being subjected to the coupling procedure or in some cases used as the salt.

### Reaction scheme 13:

### B-C Moiety Formation

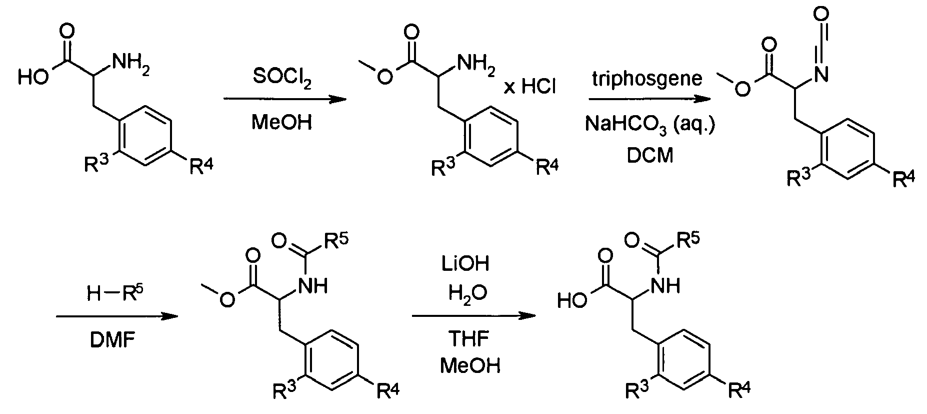

The B-C moieties can be synthesized as shown in Reaction scheme 13. Optionally substituted phenylalanine can be converted to the corresponding methyl ester hydrochloride using an activating reagent such as thionyl chloride or oxalyl chloride in methanol. Amino acid methyl ester hydrochloride can be reacted with a reagent such as triphosgene in the presence of a base such as NaHCO₃ (aq.) in a suitable solvent such as DCM to yield the isocyanate which can subsequently be reacted with an alcohol R⁵-H in a suitable solvent such as DMF. The ester function can be hydrolyzed with a base such as LiOH in a suitable solvent or solvent mixture such as water/THF/methanol to give access to the B-C-moiety.

### Reaction scheme 14:

### Alternative Reaction Sequence for B-C Moiety Formation

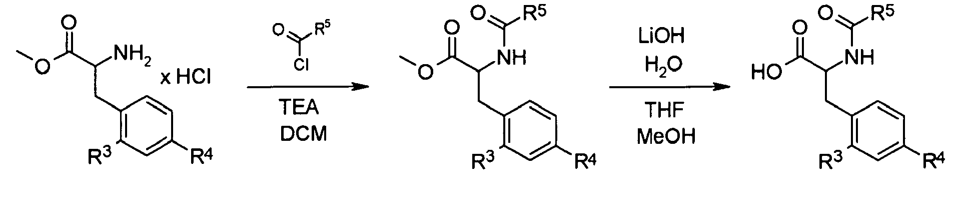

Alternatively, B-C-moieties can be obtained by reacting an optionally substituted phenylalanine methyl ester hydrochloride with an optionally substituted chloroformate ClC(O)R⁵ in the presence of a base such as triethylamine or N-methylmorpholine in a suitable solvent like DCM or THF, as is shown in Reaction scheme 14. Hydrolysis of the ester function can be accomplished as described in Reaction scheme 13.

### Reaction scheme 15:

### Coupling of A Moiety with B-C Moiety and Salt Formation

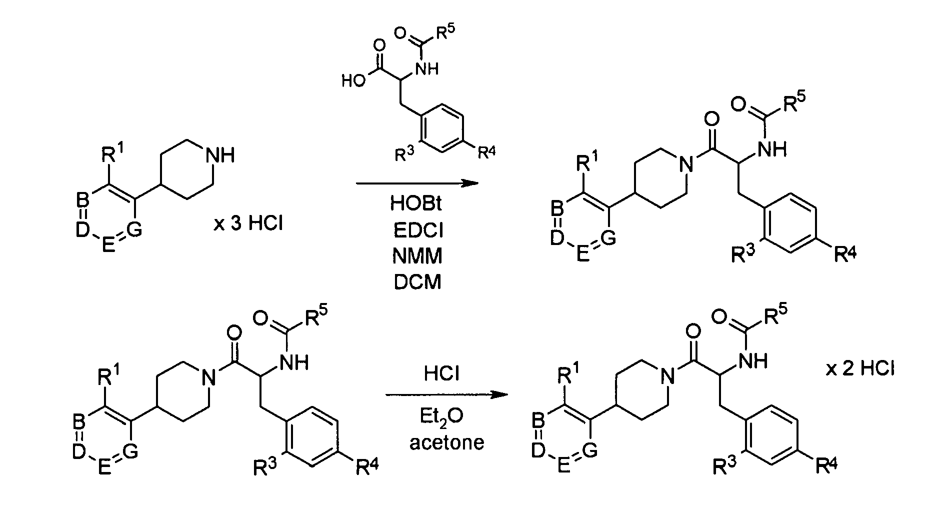

As shown in Reaction scheme 15, A moieties can be coupled with B-C moieties in the presence of EDCI/HOBt, a base such as N-methylmorpholine (NMM) and a solvent such as dichloromethane (DCM). A suitable solvent, such as DCM, DMF, THF or a mixture of the above solvents, can be used for the coupling procedure. A suitable base includes triethylamine (TEA), diisopropylethylamine (DIEA), N-methylmorpholine (NMM), collidine or 2,6-lutidine. A base may not be needed when EDCI/HOBt is used.

Generally after the reaction is completed, the reaction mixture can be diluted with an appropriate organic solvent, such as EtOAc, DCM or Et₂O, which is then washed with aqueous solutions, such as water, HCl, NaHSO₄, bicarbonate, NaH₂PO₄, phosphate buffer (pH 7), brine or any combination thereof. The reaction mixture can be concentrated and then be partitioned between an appropriate organic solvent and an aqueous solution. The reaction mixture can be concentrated and subjected to chromatography without aqueous workup.

The product can be transferred to a pharmaceutically acceptable salt such as a hydrochloride, using HCl in a solvent or solvent mixture such as diethyl ether/acetone.

### Reaction scheme 16:

### Carbamate Formation on A-B Intermediate

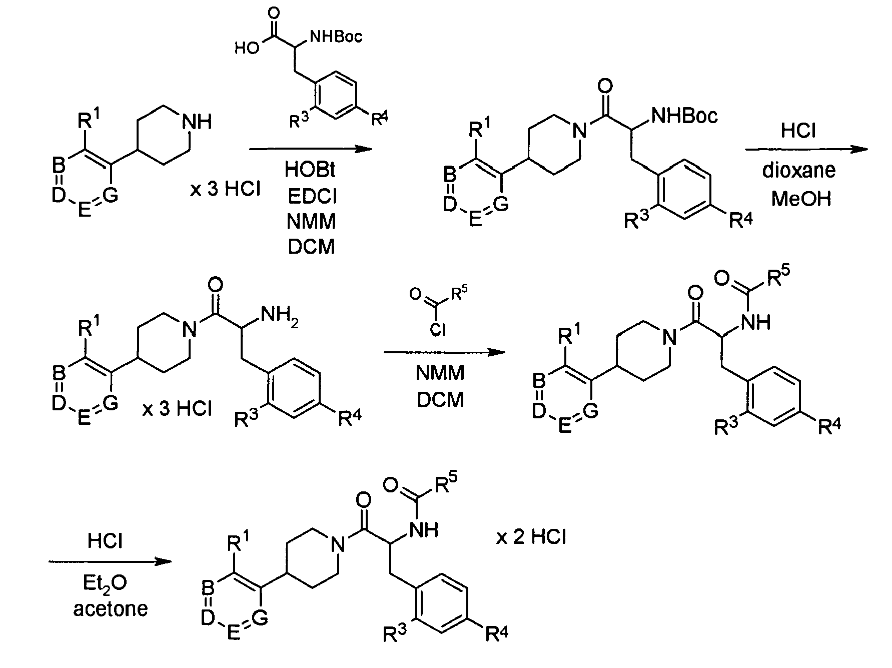

The three moieties can also be combined stepwise, as shown in Reaction scheme 16. An appropriate A moiety is coupled to a Boc-protected B moiety in the presence of EDCI/HOBt, a base such as N-methylmorpholine (NMM) and a solvent such as dichloromethane (DCM) followed by Boc deprotection with the aid of hydrogen chloride in a mixture of dioxane and methanol. The product can be reacted with an optionally substituted chloroformate in the presence of a base such as NMM or TEA in an appropriate solvent such as DCM or THF to yield the target compound. The final product can be converted to a pharmaceutically acceptable salt as described above.

### Analytical LC-MS

The compounds of the present invention according to formula (I) were analyzed via analytical LC-MS. The conditions used in the analysis are summarized below.

### Analytical conditions summary:

LC10Advp-Pump (Shimadzu) with SPD-M10Avp UV/Vis diode array detector and QP2010 MS-detector in ESI+ modus with UV-detection at 214 and 254 nm,
Column: Waters XTerra MS C18, 3.5 µm, 2.1 * 100 mm,
linear gradient with acetonitrile in water (0.15% HCOOH)
Flow rate of 0,4 ml/min;

| | |
|---|---|
| Mobile Phase A: | water (0.15% HCOOH + 5% acetonitrile) |
| Mobile Phase B: | acetonitrile (0.15% HCOOH + 5% water) |

### Gradient A:

start concentration 10% acetonitrile (0.15% HCOOH)

| | | |
|---|---|---|
| 10.00 min | B.Conc | 60 |
| 11.00 min | B.Curve | 2 |
| 12.00 min | B.Conc | 99 |
| 15.00 min | B.Conc | 99 |
| 15.20 min | B.Conc | 10 |
| 18.00 min | Pump | STOP |

The following tables describe detailed examples of the invention which can be prepared according to the Reaction schemes 1 to 16. These examples are, however, not construed to limit the scope of the invention in any manner.

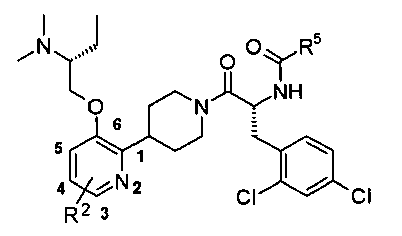

**Table 1:**

| | | | | HPLC | | MS | |
|---|---|---|---|---|---|---|---|
| No. | salt | R⁵ | R² | t_{R} (min) | method | MW (calc.) free base | [M+H]⁺ (found) |
| 1 | HCOOH | 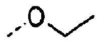 | H | 4.82 | A | 565.54 | 566 |
| 2 | HCOOH | 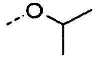 | H | 5.10 | A | 579.57 | 579 |
| 3 | HCOOH | 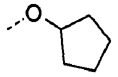 | H | 5.78 | A | 605.60 | 606 |
| 4 | 2x HCOOH | 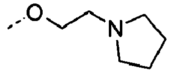 | H | 3.15 | A | 634.65 | 634 |

The following examples are provided to illustrate the invention and are not limiting the scope of the invention in any manner.

### Common Intermediates:

### 1-tert-Butoxycarbonylpiperidin-4-yl)(iodo)zinc:

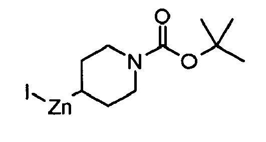

**Zinc activation**. A schlenk flask was charged with Celite (1.28 g) and dried by heating *in vacuo.* Then zinc dust (6.51 g) and dry *N,N*-dimethylacetamide (15 ml) were added. The mixture was stirred at room temperature while a 7:5 v/v mixture of chlorotrimethylsilane (1.14 ml) and 1,2-dibromoethane (0.80 ml) as solution in *N,N*-dimethylacetamide (1 ml) was added at a rate to maintain the temperature below 65°C (∼15 min). The resulting slurry was aged for 15 min.

**Zink insertion**. A solution of Boc-4-iodopiperidine (24.8 g) in *N*,*N*-dimethylacetamide (39 ml) was slowly added to the mixture described above at a rate to maintain a temperature below 65°C (∼20 min). The resulting reaction mixture was then aged for 30 min at room temperature. The suspension was filtered through a frit under argon to remove all solids. The resulting pale yellow solution was stored at room temperature under argon and was directly used for the coupling reactions.

### 3-Fluoro-3',4',5',6'-tetrahydro-2'H-[2,4']bipyridinyl-1'-carboxylic acid tert-butyl ester:

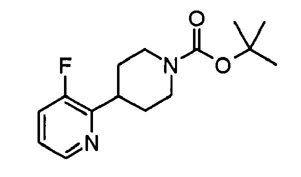

A 500 ml flask was charged with 2-bromo-3-fluoropyridine (10.0 g), [1,1'-bis(diphenylphosphino)ferrocene]dichloropalladium(II) complex with dichloromethane (1.39 g), copper(I) iodide (0.65 g), and dry *N*,*N*-dimethylacetamide (80 ml). The resulting mixture was degassed with alternating vacuum/argon purges. Then 1-tert-butoxycarbonylpiperidin-4-yl)(iodo)zinc (79.7 mmol) was added. The mixture was degassed once again and then heated to 80°C overnight. The main part of *N,N-*dimethylacetamide was then evaporated and the remainder was taken up in a mixture of EtOAc and water (500 ml each). This was then filtered through Celite and transferred into a separatory funnel. The phases were separated and the water layer was extracted with EtOAc (3 x 250 ml). The combined organic layer was washed with water and brine (500 ml each), dried (Na₂SO₄), filtered and concentrated *in vacuo.* The crude product was purified by chromatography to furnish the desired compound in form of a brown solid.

### (R)-2-Dimethylamino-butan-1-ol:

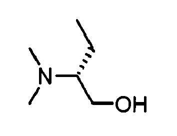

(R)-(-)-2-Amino-1-butanol (5.00 g) was added dropwise to cooled formic acid (11.0 ml). Formaldehyde (36.5% aq., 10.73 ml) was added and the mixture was stirred at 0°C for 1 h. It was then heated to 90°C for 4 h. The reaction was evaporated *in vacuo* and the oily residue was partitioned between DCM (150 ml) and 1 N NaOH. The organic phase was washed with 1 N Na₂CO₃ and brine. The DCM phase was dried over MgSO₄, filtered and evaporated to yield a yellow oil. It was purified by Kugelrohr distillation (60 mbar, 85-130°C). The product was obtained in form of a colorless oil.

### 3-((R)-2-Dimethylamino-butoxy)-3',4',5',6'-tetrahydro-2'H-[2,4']bipyridinyl-1'-carboxylic acid tert-butyl ester:

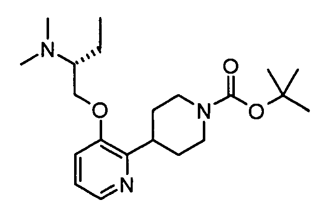

(R)-2-Dimethylamino-butan-1-ol (1.004 g) was added under argon at 0°C to a suspension of sodium hydride (60% in mineral oil, 240 mg) in DMF (30 ml). The cooling bath was removed and the mixture was stirred at room temperature for 2 h. Then 3-fluoro-3',4',5',6'-tetrahydro-2'H-[2,4']bipyridinyl-1'-carboxylic acid tert-butyl ester (1.200 g) was added and the reaction mixture was heated to 120°C (oil bath temperature). After being stirred at 120°C overnight the reaction mixture was concentrated *in vacuo.* The remaining dark brown oil was dissolved in ethyl acetate (150 ml) and the mixture was washed with 1 N Na₂CO₃ (2 x 60 ml). The combined aqueous layer was re-extracted with EtOAc (30 ml) and the combined organic layer was washed with brine (50 ml), dried (MgSO₄), filtered and evaporated to yield a dark brown oil. It was purified by column chromatography.

### [(R)-1-(1',2',3',4',5',6'-Hexahydro-[2,4']bipyridinyl-3-yloxymethyl)-propyl]-dimethylamine trihydrochloride:

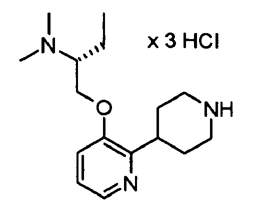

3-((R)-2-Dimethylamino-butoxy)-3',4',5',6'-tetrahydro-2'H-[2,4']bipyridinyl-1'-carboxylic acid tert-butyl ester (788 mg) was dissolved in a mixture of dioxane (15 ml) and methanol (4 ml). 4 N HCl in dioxane (15 ml) was added and the reaction was stirred at room temperature for 1 h. Evaporation of all volatiles including co-evaporation with toluene (4 x 30 ml) gave a beige semisolid, which was dried further in high vacuum overnight. The remaining solid was triturated with 10 ml of dry diethyl ether. The solvent was decanted and the product was dried *in vacuo.*

### Synthesis of B-C Moiety Precursors:

### Intermediate A1):

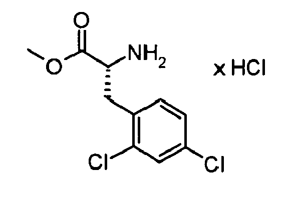

To a suspension of D-2,4-dichlorophenylalanine (10.00 g) in methanol (100 ml) was dropwise added thionylchloride (9.39 ml). During the course of the addition a clear solution was formed and the reaction started to reflux. The reaction mixture was kept under reflux for 2 h. After cooling to room temperature the mixture was evaporated to dryness at 40°C. The crude product was triturated in diethyl ether, and the insoluble compound was filtered off, washed with diethyl ether, and finally dried *in vacuo* at room temperature over P₂O₅ overnight. The product was obtained in form of colorless needles.

### Intermediate B1):

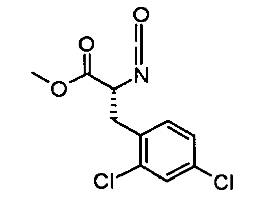

A 350 ml three-necked, flat-bottomed flask was equipped with a mechanical stirrer and charged with DCM (80 ml), saturated aqueous sodium bicarbonate solution (80 ml), and intermediate A1) (5.69 g). The biphasic mixture was cooled in an ice bath and stirred mechanically while triphosgene (1.96 g) was added in a single portion. The reaction mixture was stirred in the ice bath for 45 min and then poured into a 250 ml separatory funnel. The organic layer was collected, and the aqueous layer was extracted with three 20 ml portions of DCM. The combined organic layer was washed with water, dried over Na₂SO₄, filtered, and evaporated *in vacuo* to dryness to yield the crude product as a semisolid. The residue was purified by Kugelrohr distillation (200-240°C, 0.04-0.08 mbar). The product was obtained as clear colorless oil.

### Synthesis of Example 1:

### Intermediate 1a):

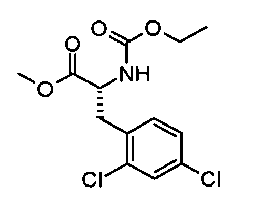

To a suspension of Intermediate A1) (1.00 g) in DCM (40 ml) was added TEA (2.47 ml). The mixture was cooled in an ice bath while stirring. Ethyl chloroformate (502 µl) was added and the reaction mixture was stirred at 0°C for 60 min. The ice bath was removed and stirring was continued at RT over night and at reflux temperature for additional 3 h. The reaction mixture was evaporated, the residue diluted with EtOAc and washed with sat. aqueous NaHCO₃ solution, 0.1 N aqueous HCl, water and brine. The aqueous layer was re-extracted with EtOAc. The combined organic layer was dried over Na₂SO₄ and the solvent was removed under reduced pressure. The crude product was purified by column chromatography to yield a white powder.

### Intermediate 1b):

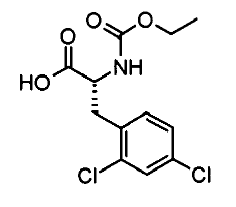

Intermediate 1a) (684 mg) was dissolved in MeOH (5 ml) and THF (18 ml) at 0°C. A solution of the lithium hydroxide monohydrate (143 mg) in water (5 ml) was added. The mixture was stirred at 0°C for 3 h. The reaction mixture was neutralized by addition of 1 N HCl (1 ml) and volatiles were evaporated *in vacuo.* The aqueous phase was acidified with 1 N HCl (pH ∼1-2). A white suspension formed. The aqueous phase was extracted twice with EtOAc (∼80 ml each). The organic layer was washed with water and brine. The aqueous phases were re-extracted with EtOAc, the combined organic layer was dried over Na₂SO₄ and evaporated *in vacuo* to yield an off-white foam.

### Example 1:

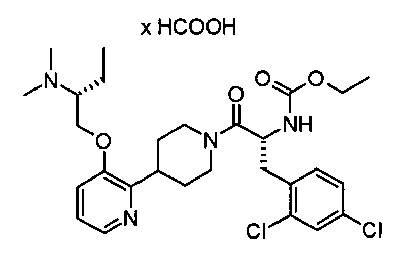

[(R)-1-(1',2',3',4',5',6'-Hexahydro-[2,4']bipyridinyl-3-yloxymethyl)-propyl]-dimethylamine trihydrochloride (266 mg), intermediate 1b) (274 mg) and HOBt (158.1 mg) were dissolved in DCM (12 ml). EDCI (158 mg) and NMM (227 µl) were added and the mixture was stirred at room temperature for one hour. A second portion of NMM (76 µl) was added and stirring was continued over night. DCM was evaporated *in vacuum* and the residue was dissolved in ethyl acetate (50 ml). The organic solution was washed with 1 N Na₂CO₃ (3 x 30 ml) and brine. Each aqueous phase was re-extracted once with ethyl acetate. The combined organic layer was dried (Na₂SO₄), filtered and evaporated *in vacuo.* The crude product was purified by preparative HPLC.

### Synthesis of Example 2:

### Intermediate 2a):

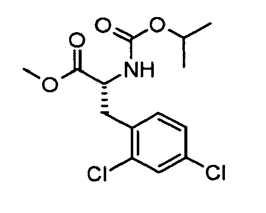

To a suspension of Intermediate A1) (1.00 g) in DCM (40 ml) was added TEA (2.47 ml). The mixture was cooled in an ice bath while stirring. Isopropyl chloroformate (1 M solution in toluene, 8.79 ml) was added and the reaction mixture was stirred at 0°C for 60 min. The ice bath was removed and stirring was continued at RT for 3 d. The reaction mixture was evaporated, the residue diluted with EtOAc and washed with sat. aqueous NaHCO₃ solution, 0.1 N aqueous HCl, water and brine. Each aqueous layer was re-extracted with EtOAc. The combined organic layer was dried over Na₂SO₄ and the solvent was removed under reduced pressure. The crude product was purified by column chromatography to yield a white powder.

### Intermediate 2b):

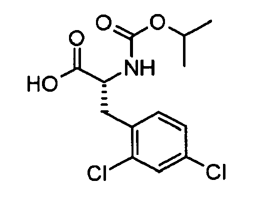

Intermediate 2a) (580 mg) was dissolved in MeOH (5 ml) and THF (18 ml) at 0°C. A solution of the lithium hydroxide monohydrate (117 mg) in water (5 ml) was added. The mixture was stirred at 0°C for 3 h. The reaction mixture was neutralized by addition of 1 N HCl (pH ∼6) and volatiles were evaporated *in vacuo.* The aqueous phase was acidified with 1 N HCl (pH ∼1-2). A white suspension formed. The aqueous phase was extracted twice with EtOAc. The organic layer was washed with water and brine. The aqueous phases were re-extracted with EtOAc. The combined organic layer was dried over Na₂SO₄ and evaporated *in vacuo* to yield a white powder.

### Example 2:

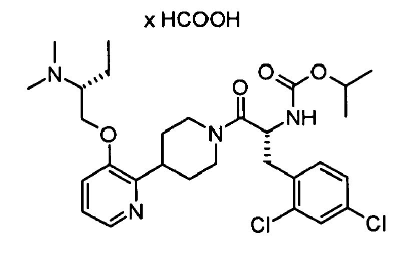

[(R)-1-(1',2',3',4',5',6'-Hexahydro-[2,4']bipyridinyl-3-yloxymethyl)-propyl]-dimethylamine trihydrochloride (233 mg), intermediate 2b) (250 mg) and HOBt (138 mg) were dissolved in DCM (12 ml). EDCI (138 mg) and NMM (198 µl) were added and the mixture was stirred at room temperature for one hour. A second portion of NMM (66 µl) was added and stirring was continued over night. DCM was evaporated *in vacuum* and the residue was dissolved in ethyl acetate (50 ml). The organic solution was washed with 1 N Na₂CO₃ (3 x 30 ml) and brine. Each aqueous phase was re-extracted once with ethyl acetate. The combined organic layer was dried (Na₂SO₄), filtered and evaporated *in vacuo.* The crude product was purified by preparative HPLC.

### Synthesis of Example 3:

### Intermediate 3a):

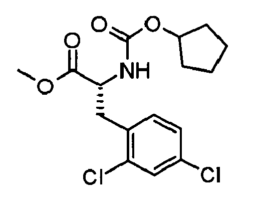

To a suspension of Intermediate A1) (500 mg) in THF (20 ml) was added TEA (1235 µl). The mixture was cooled in an ice bath while stirring. Cyclopentyl chloroformate (653 mg) was added and the reaction mixture was stirred at 0°C for 60 min. The ice bath was removed and stirring was continued at RT overnight. The reaction mixture was evaporated, the residue diluted with EtOAc and washed with sat. aqueous NaHCO₃ solution, 0.1 N aqueous HCl, water and brine. Each aqueous layer was re-extracted with EtOAc. The combined organic layer was dried over Na₂SO₄ and the solvent was removed under reduced pressure. The product was obtained in form of a white powder.

### Intermediate 3b):

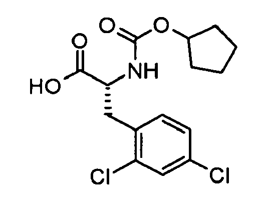

Intermediate 3a) (633 mg) was dissolved in MeOH (5 ml) and THF (18 ml) at 0°C. A solution of the lithium hydroxide monohydrate (118 mg) in water (5 ml) was added. The mixture was stirred at 0°C for 3 h. The reaction mixture was neutralized by addition of 1 N HCl (pH ∼6) and volatiles were evaporated *in vacuo.* The aqueous phase was acidified with 1 N HCl (pH ∼1-2). A white suspension formed. The aqueous phase was extracted with EtOAc. The organic layer was washed with water and brine. The aqueous phases were re-extracted with EtOAc, the combined organic layer was dried over Na₂SO₄ and evaporated *in vacuo* to yield a white powder.

### Example 3:

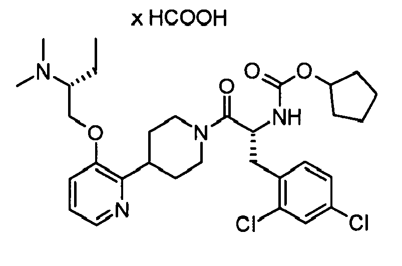

[(R)-1-(1',2',3',4',5',6'-Hexahydro-[2,4']bipyridinyl-3-yloxymethyl)-propyl]-dimethylamine trihydrochloride (215 mg), intermediate 3b) (250 mg) and HOBt (128 mg) were dissolved in DCM (12 ml). EDCI (128 mg) and NMM (183 µl) were added and the mixture was stirred at room temperature for one hour. A second portion of NMM (61 µl) was added and stirring was continued over night. DCM was evaporated *in vacuum* and the residue was dissolved in ethyl acetate (50 ml). The organic solution was washed with 1 N Na₂CO₃ (3 x 30 ml) and brine. Each aqueous phase was re-extracted once with ethyl acetate. The combined organic layer was dried (Na₂SO₄), filtered and evaporated *in vacuo.* The crude product was purified by preparative HPLC.

### Synthesis of Example 4:

### Intermediate 4a):

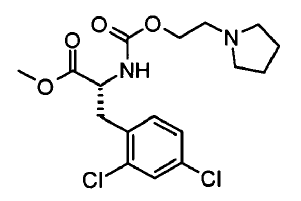

To an ice cold solution of Intermediate B1) in anhydrous DMF (10 ml) was added a solution of N-(2-hydroxyethyl)-pyrrolidine (255 µl) and triethylamine (169 µl) in anhydrous DMF (4 ml) under Ar atmosphere. The stirred mixture was warmed from 0°C to room temperature and stirring at room temperature was continued for 2 days. The reaction mixture was evaporated *in vacuo.* The residue was diluted with DCM (50 ml), the organic phase was washed with a 1 M aqueous solution of NaHCO₃ (25 ml) and brine. Each aqueous phase was re-extracted with DCM, and then the combined organic phase was dried over Na₂SO₄, filtered and evaporated. The crude product was taken up with methanol and the precipitate was filtered off. The filtrate was evaporated and the residue purified by column chromatography. The product was obtained in form of a yellow oil.

### Intermediate 4b):

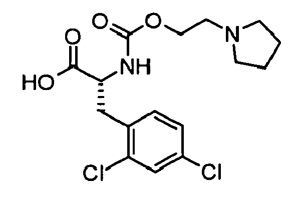

Intermediate 4a) (216 mg) was dissolved in MeOH (1.1 ml) and THF (3.0 ml) at 0°C. A solution of the lithium hydroxide monohydrate (24 mg) in water (1.1 ml) was added. The ice bath was removed and the mixture was stirred for 1 h while warming up to room temperature. Volatiles were removed and the residue was taken up in water and subjected to ion-exchange column chromatography (Separtis, Isolute SCX-2, 2 x 10 g columns). After the product was eluated with aqueous ammonia, solvents were removed under reduced pressure and the product was obtained in form of a pale yellow foam.

### Example 4:

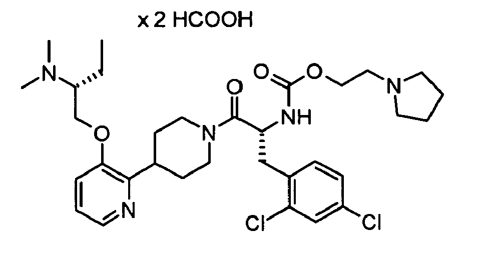

[(R)-1-(1',2',3',4',5',6'-Hexahydro-[2,4']bipyridinyl-3-yloxymethyl)-propyl]-dimethylamine trihydrochloride (143 mg), intermediate 4b) (163 mg) and HOBt (60 mg) were dissolved in DMF (4 ml). EDCI (121 mg) and NMM (89 µl) were added and the mixture was stirred at room temperature for one hour. A second portion of NMM (41 µl) was added and stirring was continued over night. DMF was evaporated *in vacuum* and the residue was dissolved in ethyl acetate (50 ml). The organic solution was washed with 1 N Na₂CO₃ (3 x 30 ml) and brine. Each aqueous phase was re-extracted once with ethyl acetate. The combined organic layer was dried (Na₂SO₄), filtered and evaporated *in vacuo.* The crude product was purified by preparative HPLC.

### Biological Assays

### A. Binding Assay

A membrane binding assay is used to identify competitive inhibitors of fluorescence labeled NDP-alpha-MSH binding to HEK293 cell membrane preparations expressing human melanocortin receptors.

The test compound or unlabeled NDP-alpha-MSH is dispensed at varying concentrations to a 384 well microtiter plate. Fluorescence labeled NDP-alpha-MSH is dispensed at a single concentration, followed by addition of membrane preparations. The plate is incubated for 5 h at room temperature.

The degree of fluorescence polarization is determined with a fluorescence polarization microplate reader.

### B. Functional Assay

Agonistic activity of human melanocortin receptors is determined in a homogeneous membrane based assay. Competition between unlabeled cAMP and a fixed quantity of fluorescence labeled cAMP for a limited number of binding sites on a cAMP specific antibody is revealed by fluorescence polarization.

The test compound or unlabeled NDP-alpha-MSH is dispensed at varying concentrations to a 384 well microtiter plate. Membrane preparations from HEK293 cells expressing the human melanocortin receptors are added. After a short preincubation period, an appropriate amount of ATP, GTP and the cAMP antibody is added and the plate is further incubated before the fluorescence labeled cAMP conjugate is dispensed. The plate is incubated for 2 h at 4°C before it is read on a fluorescence polarization microplate reader. The amount of cAMP produced as a response to a test compound is compared to the production of cAMP resulting from stimulation with NDP-alpha-MSH.

The compounds of the present invention were tested and found to bind to the melanocortin-4 receptor. The compounds were generally found to have IC₅₀ values less than 2 µM.

**Table 2: Biological data for selected examples of the invention**

| | | | |
|---|---|---|---|
| In the table are listed the IC₅₀ values of the hMC-4R binding assay and the EC₅₀ values of the functional assay. The IC₅₀ and EC₅₀ values were grouped in 3 classes: a ≤ 0.1 µM; b > 0.1 µM and ≤ 1.0 µM; c > 1.0 µM | | | |

| Example | hMC-4R binding assay IC₅₀/µM | hMC-4R functional assay EC₅₀/µM | % activation functional assay |
|---|---|---|---|
| SHU-9119 | a | - | 7 |
| NDP-α-MSH | a | a | 100 |
| 1 | a | - | -6 |
| 2 | a | - | 4 |
| 3 | b | - | 0 |

### C. In Vivo Food Intake Models

### 1. Spontaneous Feeding Paradigm

Food intake in rats is measured after i.p., s.c. or p.o. administration of the test compound (see e.g. A.S. Chen et al. Transgenic Res 2000 Apr ;9(2):145-154).

### 2. Model of LPS-Induced Anorexia and Tumor-Induced Cachexia

Prevention or amelioration of anorexia induced by either lipopolysaccharide (LPS) administration or cachexia induced by tumor growth is determined upon i.p. or p.o. administration of test compounds to rats (see e.g. D.L. Marks, N. Ling, and R.D. Cone, Cancer Res 2001 Feb 15;61 (4):1432-1438).

### D. In Vivo Model for Depression

### Forced Swim Test in Mice

### Principle

Animals placed in a container filled with water show periods of increased swimming activity and periods of relative immobility. Clinically active anti-depressants have been found to delay the onset of the first phase of immobility and to reduce the total time of relative immobility. The list of active compounds includes monoamino-oxidase-A (MAO-A) inhibitors such as moclobemide, brofaromine, noradrenaline (NA) uptake inhibitors such as imipramine and amytryptilin, MAO-B inhibitors such as selegiline and tranylcypromine, serotonin uptake inhibitors (SSRI) such as fluoxetine and paroxetine and combined NA /SSRI such as venlafaxine. Benzodiazepines and other types of psychoactive compounds have been found to be inactive in this test (see e.g. Porsolt R.D., Bertin A., Jaffre M. Behavioral despair in rats and mice: strain differences and the effect of imipramine. Eur. J. Pharmacol. 1978, 51: 291-294, Borsini F. and Meli A. Is the forced swimming test a suitable model for revealing antidepressant activity (Psychopharmacol. 1988, 94: 147-160).

### Experimental Procedure

Subjects to be used are male Swiss mice (4-5 weeks old). Animals are randomly assigned to different groups (10 mice per group).

Each animal is placed individually in the water bath where it remains for 6 minutes. The animal is given an accommodation period of 2 minutes. During the subsequent 4 minutes observation period, the duration of the periods of immobility is recorded. In addition, the frequency of the immobility state is also measured. The mouse is considered to be immobile when it passively floats on the water making only small movements to keep its head above the surface.

The water is replaced with clean water after 3 animals tested.

### Drug Administration

Treatment is administered before the test as vehicle or test compound at different doses. Compounds are usually administered by p.o. i.p. or s.c. routes.

### Data Analysis

Analysis of data is performed using ANOVA followed by Fisher's PLSD test as post-hoc test.

### Examples of a Pharmaceutical Composition

As a specific embodiment of an oral composition of a compound of the present invention, 29 mg of Example 1 is formulated with sufficient finely divided lactose to provide a total amount of 580 to 590 mg to fill a size 0 hard gelatin capsule.

While the invention has been described and illustrated in reference to certain preferred embodiments thereof, those skilled in the art will appreciate that various changes, modifications and substitutions can be made therein without departing from the spirit and scope of the invention. For example, effective dosages, other than the preferred doses as set forth above, may be applicable as a consequence of the specific pharmacological responses observed and may vary depending upon the particular active compound selected, as well as from the type of formulation and mode of administration employed, and such expected variations or differences in the results are contemplated in accordance with the objects and practices of the present invention. It is intended, therefore, that the invention be limited only by the scope of the claims which follow and that such claims be interpreted as broadly as is reasonable.

## Claims

1. A compound according to formula (I) 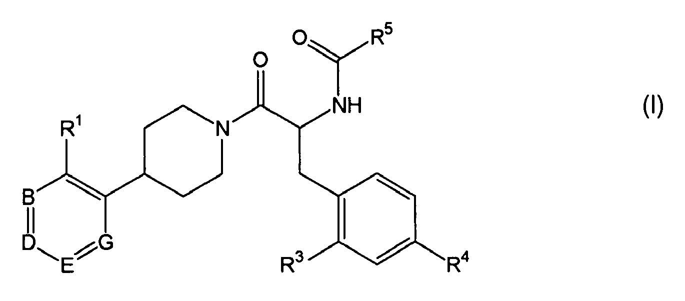 and the enantiomers, diastereomers, tautomers, solvates and pharmaceutically acceptable salts thereof,
wherein
R¹ is -N(R¹⁰)-(C(R⁶)₂)ₘ-T
-(C(R⁶)₂)ₗ-T, or
-O-(C(R⁶)₂)ₘ-T;
R⁶ is independently selected from
H,
F,
OH,
OCH₃,
C₁₋₆-alkyl, optionally substituted with 1 to 3 substituents selected from halogen, CN, OH and OCH₃, and
C₃₋₆-cycloalkyl, optionally substituted with 1 to 3 substituents selected from halogen, CN, OH and OCH₃;
T is NR⁷R⁸, 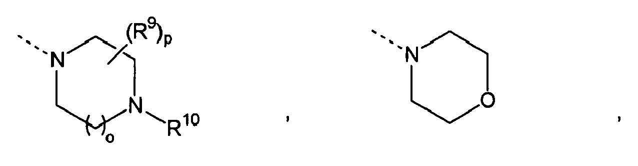 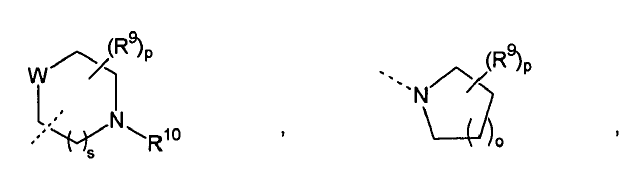 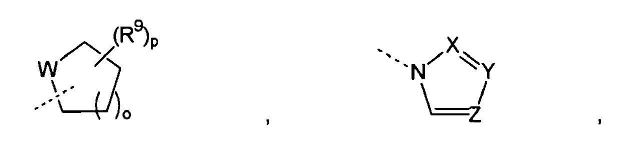 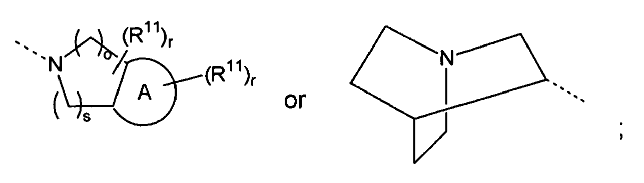
R⁷ and R⁸ are independently from each other selected from
H,
C₁₋₆-alkyl,
C₂₋₆-alkenyl,
C₂₋₆-alkinyl, and
C₂₋₆-alkylene-O-C₁₋₆-alkyl,
wherein each alkyl, alkenyl and alkinyl is optionally substituted by one or more halogen atoms, CN or OH;
R⁹ is independently selected from
halogen,
CN,
OH,
C₁₋₆-alkyl, optionally substituted with 1 to 3 substituents selected from halogen, CN and OH, and
O-C₁₋₆-alkyl, optionally substituted with 1 to 3 substituents selected from halogen, CN and OH,
C₁₋₆-alkylene-O-C₁₋₆-alkyl, optionally substituted with 1 to 3 substituents selected from halogen, CN and OH, or
NR¹²R¹³;
R¹⁰ is H, or
C₁₋₆-alkyl;
R¹¹ is independently selected from
halogen,
CN,
OH,
C₁₋₆-alkyl, optionally substituted with 1 to 3 substituents selected from halogen, CN and OH,
C₂₋₆-alkenyl,
C₂₋₆-alkinyl,
O-C₁₋₆-alkyl, optionally substituted with 1 to 3 substituents selected from halogen, CN and OH,
C₁₋₆-alkylene-O-C₁₋₆-alkyl, optionally substituted with 1 to 3 substituents selected from halogen, CN and OH,
C₀₋₆-alkyl-C₃₋₆-cycloalkyl,
-OC(O)C₁₋₆-alkyl,
-NH₂,
-NH(C₁₋₆-alkyl), and
-N(C₁₋₆-alkyl)₂;
R¹² and R¹³ are independently from each other selected from
C₁₋₆-alkyl, optionally substituted with OH,
C₂₋₆-alkenyl,
C₂₋₆-alkinyl,
C₂₋₆-alkylene-O-C₁₋₆-alkyl, and
C₂₋₆-alkylene-N-(C₁₋₆-alkyl)₂;
W is CH, O or NR¹⁰;
X is CH or N;
Y is CH or N;
Z is CH or N;
A is a 3-7-membered saturated, unsaturated or aromatic ring containing 0-2 nitrogen atoms;
B is CR² or N;
G is CR² or N;
D is CR² or N;
E is CR² or N;
with the proviso that one or two of the variables B, G, D and E must be N;
R² is independently selected from
H,
F,
Cl,
CH₃,
OCH₃, and
CF₃;
R³ is H,
Cl,
F, or
CH₃;
R⁴ is Cl,
F or
CH₃;
R⁵ is -O-(CH₂)ₜ-Q or -O-R¹⁵;
Q is a 3-6-membered, saturated or partially unsaturated carbocycle or heterocycle containing in the ring a nitrogen or an oxygen atom, wherein the carbocycle or hetercycle is optionally substituted by 1 to 4 same or different substitutents R¹⁴;
R¹⁴ is selected from
halogen,
CN,
OH,
C₁₋₆-alkyl, optionally substituted with 1 to 3 substituents selected from halogen, CN and OH,
C₂₋₆-alkenyl,
C₂₋₆-alkinyl,
O-C₁₋₆-alkyl, optionally substituted with 1 to 3 substituents selected from halogen, CN and OH,
C₁₋₆-alkylene-O-C₁₋₆-alkyl, optionally substituted with 1 to 3 substituents selected from halogen, CN and OH,
C₀₋₆-alkyl-C₃₋₆-cycloalkyl,
-OC(O)C₁₋₆-alkyl,
-C₀₋₆-NH₂,
-C₀₋₆-NH(C₁₋₆-alkyl), and
-C₀₋₆-N(C₁₋₆-alkyl)₂;
R¹⁵ is selected from
C₁₋₆ alkyl, optionally substituted with OH or halogen,
C₁₋₆-alkylene-O-C₁₋₆-alkyl, and
C₁₋₆-alkylene-N(C₁₋₆-alkyl)₂;
I is 0, 1, 2, 3, or 4;
m is 0, 1, 2, 3, or 4;
o is 0, 1, or 2;
p is 0, 1, 2, 3, or 4;
r is 0, 1, 2, 3, or 4;
s is 1, or 2 and
t is 0, 1 or 2.

2. The compound of claim 1 according to formula (I') 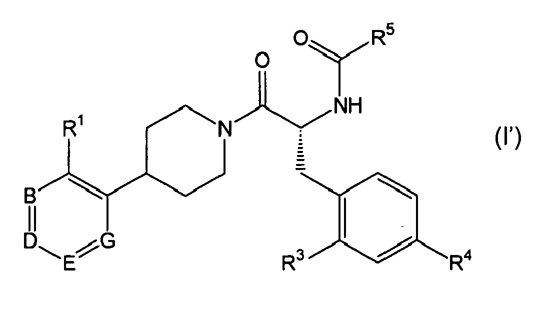 wherein B, G, D, E, R¹, R³, R⁴ and R⁵ are as defined in claim 1.

3. The compound of any of claims 1 and 2, wherein at least one of R⁷ and R⁸ is selected from
C₂₋₆-alkenyl,
C₂₋₆-alkinyl, and
C₂₋₆-alkylene-O-C₁₋₆-alkyl.

4. The compound of any of claims 1 to 3, wherein
R² is selected from H, F, Cl and CH₃.

5. The compound of any of claims 1 to 4 wherein
I is 2 or 3, or
m is 2 or 3.

6. The compound of any of claims 1 to 5 as a medicament.

7. The compound of any of claims 1 to 5 as a melanocortin-4 receptor antagonist.

8. A compound of any of claims 1 to 5 for use in the prophylaxis or treatment of a disorder, disease or condition responsive to the inactivation of the melanocortin-4 receptor in a mammal.

9. The compound of claim 8, wherein the disorder, disease of condition is selected from cachexia, muscle wasting, anorexia, anxiety, depression, amyotrophic lateral sclerosis (ALS), pain, nausea and emesis.

10. The compound of claim 9, wherein cachexia is selected from cancer cachexia, cachexia induced by chronic kidney disease (CKD) or cachexia induced by chronic heart failure (CHF).

11. The compound of claim 9, wherein the anorexia is selected from anorexia nervosa or anorexia induced by radiotherapy or chemotherapy.

12. The compound of claim 9, wherein the pain is neuropathic pain.

13. Use of the compound of any of claims 1 to 5 for the preparation of a medicament for the prophylaxis or treatment of disorders, diseases or conditions responsive to the inactivation of the melanocortin-4 receptor in a mammal.

14. The use of claim 13, wherein the disorder, disease of condition is selected from cachexia, muscle wasting, anorexia, anxiety, depression, amyotrophic lateral sclerosis (ALS), pain, nausea and emesis.

15. A pharmaceutical composition comprising a compound of any of claims 1 to 5 and a pharmaceutically acceptable carrier.
